(19)

Europäisches
Patentamt
European
Patent Office
Office européen
des brevets

(11) **EP 4 392 177 B1**

(12) **EUROPEAN PATENT SPECIFICATION**

(45) Date of publication and mention
of the grant of the patent:
**23.04.2025 Bulletin 2025/17**

(51) International Patent Classification (IPC):
**B01J 20/26** (2006.01) **G01N 33/569** (2006.01)
**B01J 20/28** (2006.01)

(21) Application number: **22783494.2**

(52) Cooperative Patent Classification (CPC):
**G01N 33/56983; B01J 20/268; B01J 20/28004;**
G01N 2600/00

(22) Date of filing: **20.09.2022**

(86) International application number:
**PCT/EP2022/076119**

(87) International publication number:
**WO 2023/041808 (23.03.2023 Gazette 2023/12)**

(54) **DETECTION OF SARS-COV-2**

NACHWEIS VON SARS-COV-2

DÉTECTION DE SARS-COV-2

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.09.2021 EP 21197636**

(43) Date of publication of application:
**03.07.2024 Bulletin 2024/27**

(73) Proprietor: **Tozaro Limited
Sharnbrook Bedfordshire MK44 1LQ (GB)**

(72) Inventors:
• **THOMSON, Alan**
  **Easton Huntingdon PE28 0TY (GB)**
• **JOHNSON, Rhiannon**
  **Bedford Bedfordshire MK41 7PW (GB)**
• **CZULAK, Joanna**
  **Bedford Bedfordshire MK41 7QS (GB)**
• **GUERREIRO, Antonio**
  **Cranfield, MK43 0DA (GB)**
• **GROVES, Alistair**
  **Rushden NN10 0NA (GB)**
• **CANFAROTTA, Francesco**
  **Burton Latimer Northamptonshire NN15 5GA
  (GB)**

(74) Representative: **Schlich
9 St Catherine's Road
Littlehampton
West Sussex BN17 5HS (GB)**

(56) References cited:
**WO-A1-2021/195626     US-B2- 10 189 934**

• **CENNAMO NUNZIO ET AL: "Proof of Concept for
a Quick and Highly Sensitive On-Site Detection
of SARS-CoV-2 by Plasmonic Optical Fibers and
Molecularly Imprinted Polymers", SENSORS,
vol. 21, no. 5, 1 March 2021 (2021-03-01), pages
1681, XP055900057, DOI: 10.3390/s21051681**
• **PARISI ORTENSIA ILARIA ET AL: ""Monoclonal-
type" plastic antibodies for SARS-CoV-2 based
on Molecularly Imprinted Polymers", BIORXIV, 28
May 2020 (2020-05-28), pages 1 - 13,
XP055858974, Retrieved from the Internet
<URL:https://www.biorxiv.org/content/10.1101/
2020.05.28.120709v1.full.pdf> [retrieved on
20211108], DOI: 10.1101/2020.05.28.120709**
• **CUBUK HASAN ET AL: "Computational analysis
of functional monomers used in molecular
imprinting for promising COVID-19 detection",
COMPUTATIONAL AND THEORETICAL
CHEMISTRY, vol. 1199, 16 March 2021
(2021-03-16), AMSTERDAM, NL, pages 113215,
XP055900168, ISSN: 2210-271X, DOI: 10.1016/
j.comptc.2021.113215**

EP 4 392 177 B1

- **PILETSKY STANISLAV ET AL: "Molecularly Imprinted Polymers for Cell Recognition", TRENDS IN BIOTECHNOLOGY, ELSEVIER PUBLICATIONS, CAMBRIDGE, GB, vol. 38, no. 4, 31 October 2019 (2019-10-31), pages 368 - 387, XP086090593, ISSN: 0167-7799, [retrieved on 20191031], DOI: 10.1016/J.TIBTECH.2019.10.002**
- **MALIK AIJAZ AHMAD ET AL: "Molecularly imprinted polymer for human viral pathogen detection", MATERIALS SCIENCE AND ENGINEERING C, ELSEVIER SCIENCE S.A, CH, vol. 77, 27 March 2017 (2017-03-27), pages 1341 - 1348, XP085028530, ISSN: 0928-4931, DOI: 10.1016/J.MSEC.2017.03.209**
- **LAN JUN ET AL: "Structure of the SARS-CoV-2 spike receptor-binding domain bound to the ACE2 receptor", NATURE, NATURE PUBLISHING GROUP UK, LONDON, vol. 581, no. 7807, 30 March 2020 (2020-03-30), pages 215 - 220, XP037182122, ISSN: 0028-0836, [retrieved on 20200330], DOI: 10.1038/S41586-020-2180-5**

Remarks:

The complete document including Reference Table(s) and the Sequence Listing(s) can be downloaded from the EPO website

## Description

## Introduction

The present invention relates to compounds for detection of SARS-CoV-2, to methods of producing the same and to conjugates comprising the same, and more particularly to use of the aforementioned in the detection of SARS-CoV-2.

## Background to the Invention

The outbreak in 2019 of a novel coronavirus, severe acute respiratory syndrome coronavirus 2 (SARS-CoV-2), has given rise to a pandemic which has in turn led to both a public health and economic crisis on a worldwide scale.
Although the international community quickly recognised that both diagnostic and antibody testing are key to preventing further transmission of the virus and to understanding the prevalence of the virus respectively, testing kits which are reliable, inexpensive to produce and easy to use remain highly sought after.
Regarding diagnostic testing, at present tests based on reverse transcription polymerase chain reaction (RT-PCR) are the predominant means by which healthcare providers are assessing citizens. The RT-PCR tests work by detecting viral ribonucleic acid (RNA) in a respiratory sample often obtained via a nasopharyngeal swab.
Whilst RT-PCR testing is widespread, there are problems. Conducting RT-PCR tests can be labour intensive: they require highly skilled personnel and false negative results can occur e.g. due to sampling error or improper sample handling. Indeed false positive results may also occur e.g. due to sample cross-contamination. The tests are also expensive and there is a long turn-around time (1-2 days) with respect to results.
Rapid antigen tests are also used for population screening and offer portable and rapid (15-30 min) analysis that can be performed at the convenience of the individual. However, they lack sensitivity and utilize antibodies as receptors, which can only function in narrow temperature and pH ranges, which limits their widespread use.
Molecularly imprinted polymers (MIPs) have been proposed as alternative receptors for use in the detection of SARS-CoV-2.
Cennamo et al in "Proof of Concept for a Quick and Highly Sensitive On-Site Detection of SARS-CoV-2 by Plasmonic Optical Fibers and Molecularly Imprinted Polymers" details the production of plastic optical fiber (POF) based surface plasmon resonance (SPR) sensors coupled with MIPs. The MIPs were made to the S1 subunit of the SARS-CoV-2 spike protein.
Parisi et al in ""Monoclonal-type" plastic antibodies for SARS-CoV-2 based on Molecularly Imprinted Polymers" describes the preparation of MIPs using the SARS-CoV-2 receptor binding domain (RBD) as a template.
Cubuk et al in "Computational analysis of functional monomers used in molecular imprinting for promising COVID-19 detection" describes computational studies aimed at identifying a template and suitable monomers to produce a MIP against SARS-CoV-2.
WO 2021/195626 A1 discloses the preparation of MIPs to SARS-CoV-2 wherein the whole virus is used as a template.
Methods of producing MIPs are also known - see e.g. US 10 189 934B2, Piletsky et al in "Molecularly Imprinted Polymers for Cell Recognition" and Malik et al in "Molecular Imprinted Polymer for human viral pathogen detection".
Using either the whole virus, the whole S1 subunit or the whole RBD of SARS-CoV-2 for the production of MIPs leads to significant issues regarding scalability.
Antibody tests, in contrast to RT-PCR and rapid antigen tests, are carried out in an attempt to establish whether a person may have already had the virus rather than, in the case of RT-PCR and rapid antigen tests, whether the person currently has the virus. Antibody tests are often carried out on blood samples from patients and work by detecting the presence of binding between synthetic antigens and antibodies (if present) in a patient's blood sample.
Accordingly, one aim of the present invention is to provide alternative means for detecting SARS-CoV-2. A further aim is that such means will provide an alternative to the polymerase chain reaction-based assay and rapid antigen tests currently used. An aim of specific embodiments of the invention includes providing improved detection means.

## Summary of the Invention

Accordingly, the invention provides a molecularly imprinted polymer comprising at least one recognition site that is complementary to a template molecule consisting of an amino acid sequence corresponding to a subsequence of the receptor binding domain of SARS-CoV-2 spike protein, wherein the amino acid sequence is no more than 50 amino acids in length and comprises the sequence NSNNLDSKVGG, and wherein the polymer comprises the monomers:

    (i) N-Fluoresceinyl acrylamide,
    (ii) Acrylamide,

(iii) Tert-Butyl acrylate (TBAc),
(iv) 3-O-Acryloyl-1,2:5,6-bis-O-isopropylidene-D-glucofuranose,
(v) 2,2,2-Trifluoroethyl methacrylate (CF3),
(vi) N-(3-Aminopropyl) methacrylamide hydrochloride (APMA),
(vii) Squareamide monomer 7 (SQ7) and
(viii) N,N'-Methylenebis(acrylamide) (BIS).

The invention also provides a method of preparing a molecularly imprinted polymer comprising at least one recognition site which binds SARS-CoV-2 comprising the steps of:

(a) providing a carrier substance having a template molecule consisting of an amino acid sequence corresponding to a subsequence of the receptor binding domain of SARS-CoV-2 spike protein immobilised on it so as to be exposed at a surface;
(b) providing a polymerisable composition in contact with the surface;
(c) effecting controlled polymerisation of the polymerisable composition in contact with the surface to produce a molecularly imprinted polymer; and
(d) separating the molecularly imprinted polymer from the surface and the immobilised template molecule,

wherein the amino acid sequence is no more than 50 amino acids in length and comprises the sequence NSNNLDSKVGG, and
wherein the polymerisable composition comprises the monomers:

(i) N-Fluoresceinyl acrylamide,
(ii) Acrylamide,
(iii) Tert-Butyl acrylate (TBAc),
(iv) 3-O-Acryloyl-1,2:5,6-bis-O-isopropylidene-D-glucofuranose,
(v) 2,2,2-Trifluoroethyl methacrylate (CF3),
(vi) N-(3-Aminopropyl) methacrylamide hydrochloride (APMA),
(vii) Squareamide monomer 7 (SQ7) and
(viii) N,N'-Methylenebis(acrylamide) (BIS).

Further provided is a conjugate comprising the molecularly imprinted polymer of the invention and a fluorophore.
Both the molecularly imprinted polymers of the invention and the conjugates of the invention can be used in the detection of SARS-CoV-2.

## Details of the Invention

The invention provides a molecularly imprinted polymer comprising at least one recognition site that is complementary to a template molecule consisting of an amino acid sequence corresponding to a subsequence of the receptor binding domain of SARS-CoV-2 spike protein, wherein the amino acid sequence is no more than 50 amino acids in length and comprises the sequence NSNNLDSKVGG, and wherein the polymer comprises the monomers:

(i) N-Fluoresceinyl acrylamide,
(ii) Acrylamide,
(iii) Tert-Butyl acrylate (TBAc),
(iv) 3-O-Acryloyl-1,2:5,6-bis-O-isopropylidene-D-glucofuranose,
(v) 2,2,2-Trifluoroethyl methacrylate (CF3),
(vi) N-(3-Aminopropyl) methacrylamide hydrochloride (APMA),
(vii) Squareamide monomer 7 (SQ7) and
(viii) N,N'-Methylenebis(acrylamide) (BIS).

In molecular imprinting, functional, and sometimes cross-linking, monomers are polymerised in an appropriate solvent in the presence of the compound to be imprinted. The compound to be imprinted is known as the template. During the polymerisation step, the monomers interact with the template through electrostatic, hydrophobic or other interactions leading to the formation of binding sites in the polymer, known as recognition sites, that are complementary to the template molecule. After removal of the template, the polymer matrix retains the recognition sites that are complementary to the template. Complementary in this sense means that the interacting groups in the polymer are placed such that they are in a suitable position to interact with the interacting groups in the template molecule and other molecules that contain the template molecule as a substructure, i.e. in this invention SARS-CoV-2.

The sequence of SARS-CoV-2 is known. SARS-CoV-2 makes use of a densely glycosylated spike protein to gain entry into host cells. The spike protein is a homotrimeric glycoprotein and each monomer contains two subunits, S1 and S2, which mediate membrane attachment and membrane fusion respectively. The receptor binding domain (RBD) of the S1 subunit has been shown to be involved in host cell receptor engagement.

Although the sequence of the RBD of the SARS-CoV-2 spike protein is known, and theoretically any sequence therein could be used as a template, it is advantageous for some downstream applications to select, as a template, sequences within the target which are non-conserved. This ensures that the molecularly imprinted polymer binds the desired target with high specificity.

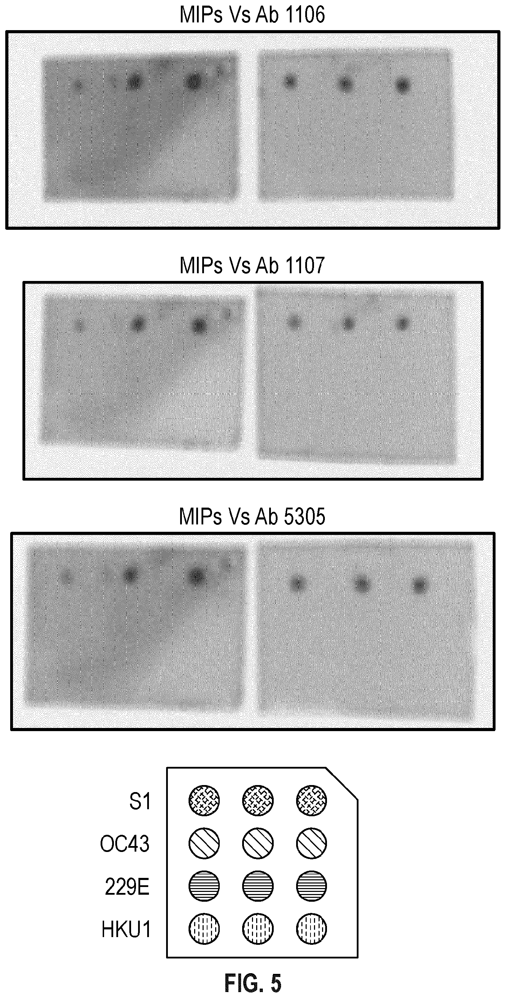

Supplementary Figure 5 of Wrapp *et al* 2020[1] presents a sequence alignment of the spike protein from SARS-CoV-2 and two other coronavirus strains, SARS-CoV and RaTG13. The RBD is highlighted. Also highlighted are sequences which are conserved between the different strains and sequences which are non-conserved.

In the present invention, the 'at least one recognition site' in the molecularly imprinted polymer is complementary to a template molecule consisting of an amino acid sequence corresponding to a subsequence of the receptor binding domain of SARS-CoV-2 spike protein, wherein the amino acid sequence is no more than 50 amino acids in length and comprises the sequence NSNNLDSKVGG.

In the present invention, the size of the template is in the range up to 50 amino acids. Preferably, the template will be less than 50 amino acids, more preferably the template will be less than 30 amino acids; most preferred is that the template is less than 20 amino acids, for example 11, 12, 13, 14, 15, 16, 17, 18 or 19 amino acids.

The use of short peptide sequences (as opposed to e.g. the whole spike protein or RBD) drastically reduces reagent costs and creates high affinity binding sites for a specific region of the target protein, therefore enhancing the monoclonality of the molecularly imprinted polymer.

The size of the molecularly imprinted polymer of the present invention will be to a large extent dictated by the intended downstream use of the molecular imprinted polymer. That said, suitably the molecular imprinted polymer will be less than 500nm, more suitably less than 250nm and even more suitably less than 100nm, and may be less than 90nm, or even less than 70nm.

Also provided by the disclosure, and not claimed, is a composition comprising the molecularly imprinted polymer of the invention. Suitably, molecular imprinted polymers in such compositions will have an average size that is less than 500nm, more suitably less than 250nm and even more suitably less than 100nm, and may be less than 90nm, or even less than 70nm.

Suitable methods for determining the size of molecularly imprinted polymers include dynamic light scattering e.g. using a Zetasizer Ultra (Malvern Panalytical), disc centrifugation, nanoparticle tracking analysis e.g. using a NanoSight NS300 (Malvern Panalytical), tunable resistive pulse sensing, atomic force microscopy and electron microscopy. Nanoparticle tracking analysis, tunable resistive pulse sensing, atomic force microscopy and electron microscopy are particularly suitable for determining the size of single molecularly imprinted polymers. In the examples provided herein, the size of the molecularly imprinted polymers was determined using a NanoSight NS300 instrument.

Size in the context of the molecularly imprinted polymer refers to the diameter of the molecularly imprinted polymer.

Monomers which can be used in the preparation of molecularly imprinted polymers include vinyl monomers, allyl monomers, acetylenes, acrylates, methacrylates, acrylamides, methacrylamides, chloroacrylates, itaconates, trifluoromethylacrylates, derivatives of amino acids (e.g. esters or amides), nucleosides, nucleotides, and carbohydrates. Cross-linking monomers, which help to stabilise molecularly imprinted polymers, may also be included. Typical examples of cross-linking monomers suitable for preparing molecularly imprinted polymers include, but are not limited to, ethylene glycol dimethacrylate, trimethylolpropane trimethacrylate, divinylbenzene, methylene bisacrylamide, ethylene bisacrylamide, N,N'-bisacryloylpiperazine and N,N'-Methylenebis(acrylamide) (BIS). Those skilled in the art could select monomers and cross-linking monomers suitable for a particular template. Alternatively a variety of combinatorial and computational methods could be used to assist in this selection.

In the invention, the molecularly imprinted polymer will comprise (i) N-Fluoresceinyl acrylamide, (ii) Acrylamide, (iii) Tert-Butyl acrylate (TBAc), (iv) 3-O-Acryloyl-1,2:5,6-bis-O-isopropylidene-D-glucofuranose, (v) 2,2,2-Trifluoroethyl methacrylate (CF3), (vi) N-(3-Aminopropyl) methacrylamide hydrochloride (APMA), (vii) Squareamide monomer 7 (SQ7) and (viii) N,N'-Methylenebis(acrylamide) (BIS). In this context it is understood that the molecularly imprinted polymer is a polymer and is said to comprise an identified monomer when it has been made by polymerising a mixture of monomers comprising the identified monomer.

Advantageously, the molecularly imprinted polymers of the present invention are robust and function in wide ranges of temperature and pH which leads to improved shelf-life and storage conditions and means that they can function in more challenging environments. They also have excellent biocompatibility and have fast binding kinetics.

Also provided by the present invention is a method of preparing a molecularly imprinted polymer comprising at least one recognition site which binds SARS-CoV-2 comprising the steps of:

(a) providing a carrier substance having a template molecule consisting of an amino acid sequence corresponding to a subsequence of the receptor binding domain of SARS-CoV-2 spike protein immobilised on it so as to be exposed at a surface;

(b) providing a polymerisable composition in contact with the surface;

(c) effecting controlled polymerisation of the polymerisable composition in contact with the surface to produce a molecularly imprinted polymer, and

(d) separating the molecularly imprinted polymer from the surface and the immobilised template molecule,

wherein the amino acid sequence is no more than 50 amino acids in length and comprises the sequence NSNNLDSKVGG, and

wherein the polymerisable composition comprises the monomers:

(i) N-Fluoresceinyl acrylamide,
(ii) Acrylamide,
(iii) Tert-Butyl acrylate (TBAc),
(iv) 3-O-Acryloyl-1,2:5,6-bis-O-isopropylidene-D-glucofuranose,
(v) 2,2,2-Trifluoroethyl methacrylate (CF3),
(vi) N-(3-Aminopropyl) methacrylamide hydrochloride (APMA),
(vii) Squareamide monomer 7 (SQ7) and
(viii) N,N'-Methylenebis(acrylamide) (BIS).

An advantage of the method is that the MIPs possess a more homogenous distribution of binding site affinities, and provides means by which template elution is enhanced and downstream template leaching is prevented.

Suitable carrier substances include polymer resins, polysaccharides, glass or metal surfaces.

The carrier substance may be in the form of beads, fibres, membranes or capillaries. In preferred embodiments of the invention, the carrier substance is glass. Further preferred is that the carrier substance is in the form of glass beads.

The polymerisable composition must contain the monomers required for the polymerisation. Suitable monomers are discussed elsewhere herein.

Step (c) involves the application of controlled polymerisation. Various techniques for producing molecularly imprinted polymers by controlled polymerisation are known to those of skill in the art.

Examples of controlled polymerisation include radical polymerisation such as controlled living radical polymerisation (LRP), living anionic polymerisation, living cationic polymerisation, and controlled polycondensation.

Polymerisation can be initiated by e.g. heating, by applying current (electropolymerisation), by the addition of redox catalyst(s), persulfate or peroxides, by irradiation, including gamma radiation or by microwave radiation or by irradiation with UV or visible light and normally takes minutes to hours. In the examples described herein, polymerisation was initiated using persulfate.

In preferred embodiments of the invention, the polymerisation reaction is terminated at a stage when the size of the synthesized molecular imprinted polymer is relatively small. Preferences with respect to size are discussed elsewhere herein.

Once the molecularly imprinted polymers are synthesized they must be separated and collected. The separation of high affinity molecularly imprinted polymers from the immobilised template can be achieved by heating, which disrupts complex formation, by changing solution pH, changing ionic strength, or through the additional of urea, guanidine or a substance which interacts with the template more strongly than the molecularly imprinted polymer.

In preferred embodiments of the method, prior to the separation step, weakly bound material is removed by washing.

Once collected, the molecularly imprinted polymers may be purified further e.g. by chromatography, filtration and/or electrophoresis.

The method by which the template molecule is immobilised on the carrier substance is not critical. However, for some methods, the presence of a cysteine residue at one of the termini of the template molecule is advantageous. As such, for embodiments of the method where the template molecule does not naturally comprise a terminal cysteine residue, it is preferred that the template molecule is modified at the N terminus to comprise an additional cysteine residue.

In some embodiments of the invention, the additional cysteine residue is attached directly to the termini of the template molecule whilst in other embodiments a spacer is included between the terminal cysteine and the template molecule. In preferred embodiments the spacer comprises a glycine residue.

Thus in a particular preferred embodiment of the present invention, the template molecule comprises the amino acid sequence NSNNLDSKVGG and is modified at the N terminus to comprise a cysteine (C) residue and a glycine (G) residue such that the molecule used for imprinting is CGNSNNLDSKVGG.

In some embodiments of the invention, the template molecule is directly attached to the carrier substance whilst in other embodiments one or more linkers are included between the carrier substance and the template molecule. Suitable linkers for use in the invention are well known to those of skill in the art. In the examples provided herein, the carrier substance used was glass beads and these were silanised using (3-aminopropyl)trimethoxysilane (APTMS) prior to immobilisation of the template molecule. The APTMS acts as a linker between the surface of the glass bead and the template molecule. In the examples provided herein, the APTMS was modified using n-succinimidyl iodoacetate (SIA) prior to template immobilisation.

Linkers and spacers function to prevent adverse effects brought about by steric hindrance.

An advantage of the method of the present invention is that it is fast and scalable and uses only a short template.

Further provided by the invention is a conjugate comprising the molecularly imprinted polymer of the invention and a fluorophore.

Fluorophores are molecules with the ability to absorb light at a particular wavelength, the absorbance or excitation wavelength, and then emit light at a higher wavelength, the emission wavelength.

Conjugation of fluorophores to molecules for visualization purposes is common in the art. As such, methods for such conjugation are also well known. By way of example, fluorophores can be conjugated via amino, thiol or carbohydrate groups. In the examples provided herein, the fluorophore was conjugated to the molecularly imprinted polymer via amine groups provided by the amino monomer in the molecularly imprinted polymer.

Although any fluorophore can be used in conjugates of the present invention, it is advantageous for some downstream applications, particularly diagnostic applications, to select a fluorophore that is both bright and stable.

Conjugated Polymer Nanoparticles (CPNs)™ are highly fluorescent nanoparticles which comprise of a semiconductor light emitting polymer (LEP) core encapsulated within a biocompatible surfactant. CPNs are particularly suited for use in diagnostic applications due to their intense fluorescence, which means they have a high level of sensitivity, and their high degree of photostability. CPNs are around 70-80nm in size and those currently available from Stream Bio exhibit fluorescence with emission wavelengths from 420nm to 1130nm. In the examples provided herein, molecularly imprinted polymers of the invention were conjugated to CPN510 and CPN610, both available from Stream Bio.

In preferred embodiments of the present invention, the fluorophore is a CPN.

Also provided by the present disclosure, and not claimed, are compositions comprising the conjugate of the invention.

The molecularly imprinted polymers and conjugates of the present invention may be used in a variety of applications.

It is specifically envisaged that the molecularly imprinted polymers and conjugates of the present invention be used in the detection of SARS-CoV-2. The molecularly imprinted polymers and conjugates and compositions may be used in assays for example diagnostic assays which may be used in a medical or point of care setting. Examples of such assays include lateral flow assays (LFA) and enzyme-linked immunosorbent assay (ELISA)-type assays which may be used for high throughput screening. The molecularly imprinted polymers and conjugates of the present invention may also be used in sensors.

In the examples provided herein, nanoMIP-SPR sensors were prepared by covalently immobilising nanoMIPs on 4% mercaptoundecanoic acid (MUDA) chips (prepared using Biacore SIA Kit Au surfaces, functionalised with a self-assembled monolayer of 4% MUDA in ethanol). NanoMIP-functionalized Screen-Printed Electrodes (SPEs) were also prepared for use in thermal detection methods.

The molecularly imprinted polymers and conjugates of the present invention may also be used in the detection of SARS-CoV-2 in the research setting.

The molecularly imprinted polymers and conjugates of the present invention may also be used as pharmaceuticals.

It is desirable that the molecularly imprinted polymers of the present invention have high affinity for SARS-CoV-2. High affinity in the context of the present specification means that the affinity with which the molecularly imprinted polymers bind SARS-CoV-2 exceeds the affinity with which 'blank' molecularly imprinted polymers bind SARS-CoV-2 by at least three, preferably at least five, more preferably at least 10 times. Blank in this context means molecularly imprinted polymers formed in the absence of template.

Binding affinity is the strength of the binding interaction between a single molecule to its binding partner e.g. in the present case a molecularly imprinted polymer to its target SARS-CoV-2. Binding affinity is typically measured and reported by the equilibrium dissociation constant ($K_D$). The smaller the $K_D$ value, the greater the binding affinity between the two molecules.

When the molecularly imprinted polymers and conjugates of the present invention are used in the detection of SARS-CoV-2, it is preferred that the molecularly imprinted polymer binds to SARS-CoV-2 with a $K_D$ of less than 500nM, preferably less than 250nM and even more preferably less than 200nM.

Suitable methods for measuring $K_D$ include surface plasmon resonance (SPR) which may be conducted using e.g. the Biacore series of instruments (Cytiva) or the MP-SPR Navi instruments (BioNavis).

## Examples

The invention is now further described in specific examples with reference to the accompanying drawings in which:

Figure 1 shows two surface plasmon resonance sensorgrams showing the binding kinetics for nanoMIPs imprinted against peptide 1 and SARS-CoV-2 S recombinant protein;

Figure 2 is a trace obtained using a NanoSight NS300 instrument showing the successful conjugation of a nanoMIP imprinted against peptide 1 to CPN510;

Figure 3 is a dot blot showing specificity of binding between nanoMIPs imprinted against peptides 1 and 3 and recombinant SARS-CoV-2 S protein;

Figure 4 is a dot blot showing specificity of binding between nanoMIPs imprinted against peptide 1 and SARS-CoV-2;

Figure 5 is a dot blot showing specificity of binding between nanoMIPs imprinted against peptide 1 and SARS-CoV-2 Spike Glycoprotein (S1);

Figure 6 is a raw Heat-Transfer Method data plot of $R_{th}$ versus time for the addition of SARS-CoV-2 ORF8 (1fg/mL - 10pg/mL) in PBS to a Screen-Printed Electrode (SPE) functionalised with NanoMIP against peptide 1 (Figure 6a) and peptide 3 (Figure 6b);

Figure 7 is a raw Heat-Transfer Method data plot of $R_{th}$ versus time for the addition of SARS-CoV-2 S protein RBD (1fg/mL - 10pg/mL) in PBS to a Screen-Printed Electrode (SPE) functionalised with NanoMIP against peptide 1 (Figure 7a) and a dose response curve whereby % change in the $R_{th}$ values were plotted against the concentration of SARS-CoV-2 S protein RBD injected into the system (Figure 7b);

Figure 8 is a raw Heat-Transfer Method data plot of $R_{th}$ versus time for the addition of SARS-CoV-2 S protein RBD (1fg/mL - 10pg/mL) in PBS to a Screen-Printed Electrode (SPE) functionalised with NanoMIP against peptide 3 (Figure 8a) and a dose response curve whereby % change in the $R_{th}$ values were plotted against the concentration of SARS-CoV-2 S protein RBD injected into the system (Figure 8b);

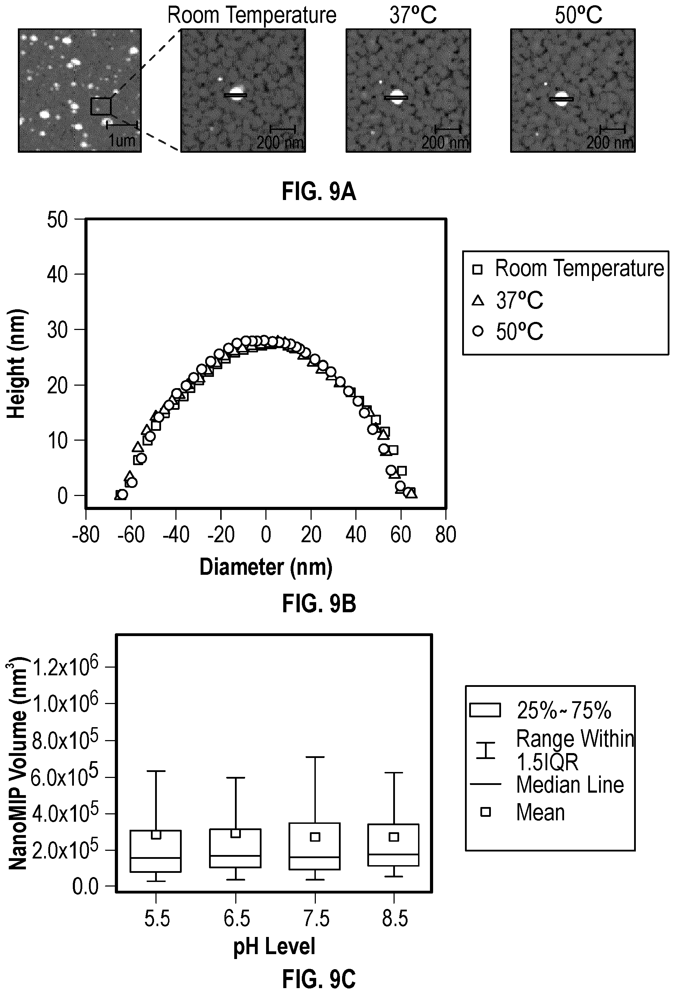

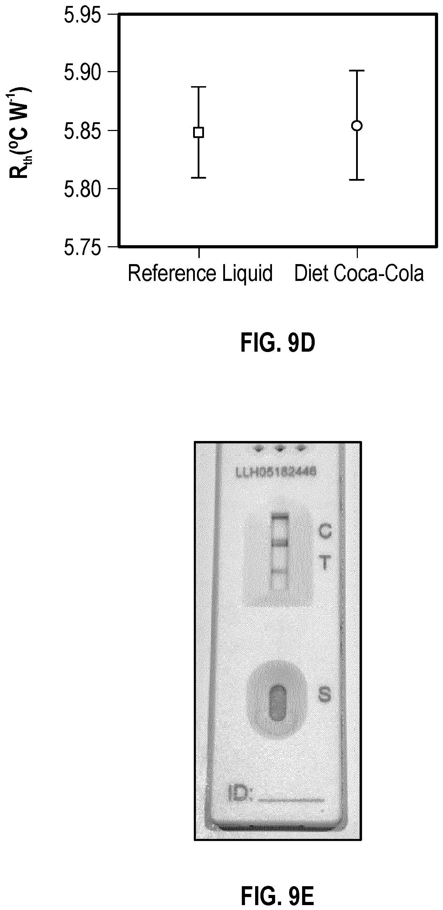

Figure 9 reports the ability of nanoMIPs imprinted against peptide 1 to withstand extremes of temperature and pH. Figure 9a shows typical atomic force microscopy (AFM) height images of an isolated nanoMIP on an Au surface in air (represented by the green box in the larger scale image) at room temperature, 37 °C, and 50 °C. Figure 9b shows a corresponding cross-sectional profile plot of the nanoMIP at each temperature. Figure 9c shows a box chart comparing nanoMIP volume (n = 120) from AFM images at various pH levels. Figure 9d compares the thermal response of a nanoMIP-based sensor to a clinical reference liquid (universal transport medium) and Diet Coca-Cola (pH = 3.5). Figure 9e shows a commercial rapid antigen test giving a false positive result when Diet Coca-Cola was used as the test liquid;

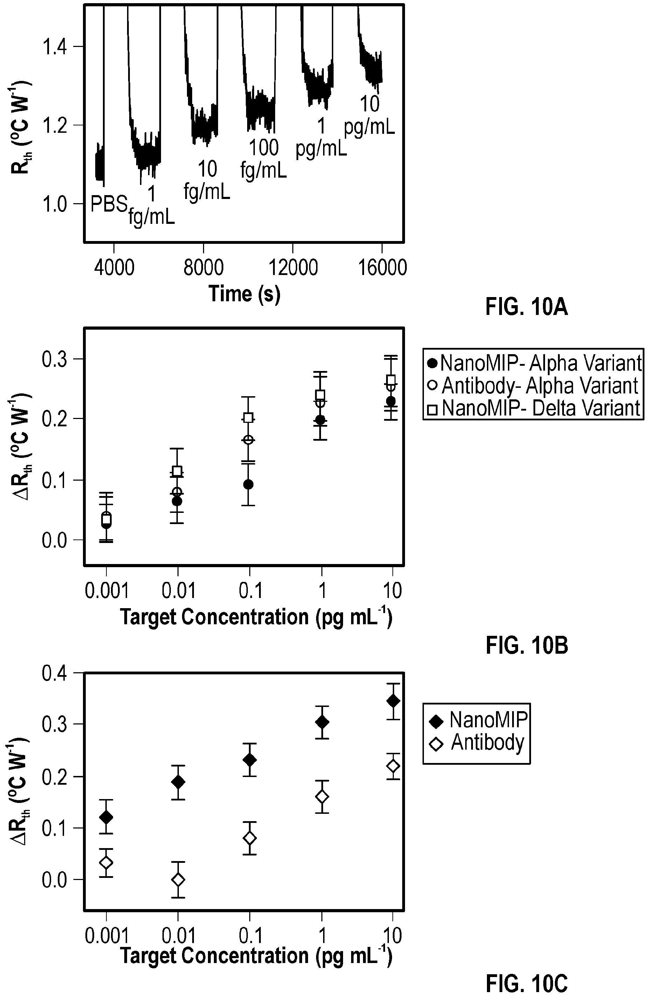

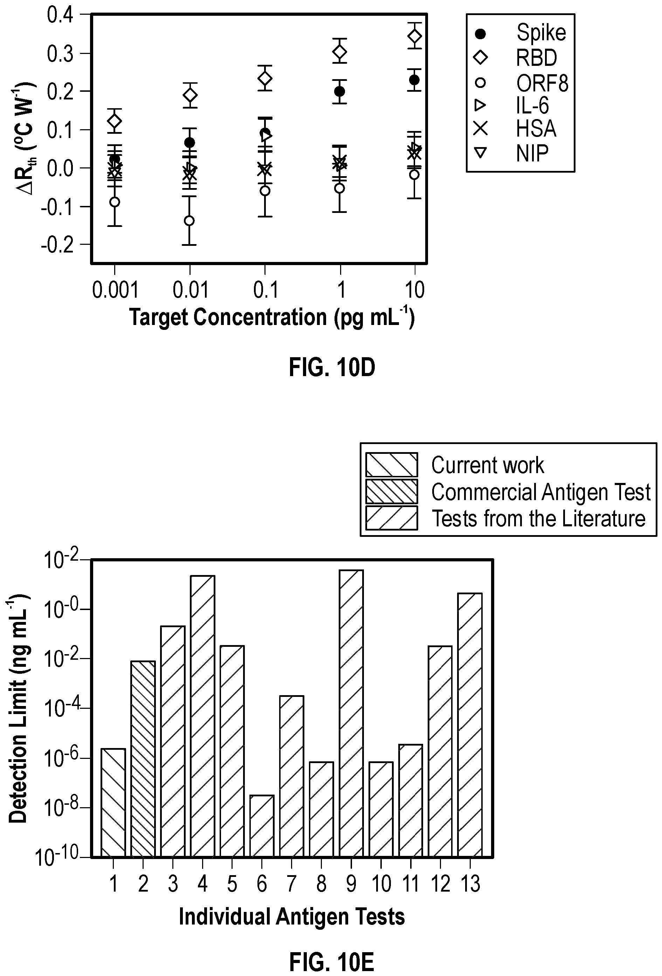

Figure 10 reports the sensitivity of nanoMIPs imprinted against peptide 1 to various SARS-CoV-2 antigens and compares the sensitivity of the nanoMIPs to a SARS-CoV-2 antibody. Figure 10a shows typical Heat Transfer Method data for a nanoMIP-functionalized SPE upon exposure to PBS containing 1 fg mL$^{-1}$ - 10 pg mL$^{-1}$ of the SARS-CoV-2 RBD. Figures 10b-10d show typical dose-response curves (error bars represent the SD) showing the thermal response of (i) nanoMIP and antibody sensors to the spike protein (nanoMIPs were tested against the SARS-CoV-2 spike protein from both the alpha and delta variants) (Figure 10b) (ii) nanoMIP and antibody sensors to the SARS-CoV-2 RBD (Figure 10c), and iii) nanoMIP sensors to SARS-CoV-2 antigens and the negative controls ORF8, IL-6, and HSA. The response of NIP-based sensors to the spike protein is also shown (Figure 10d). Figure 10e compares the LoD values for nanoMIPs of the present invention with a commercial rapid antigen test and numerous recently developed antigen tests from the literature;

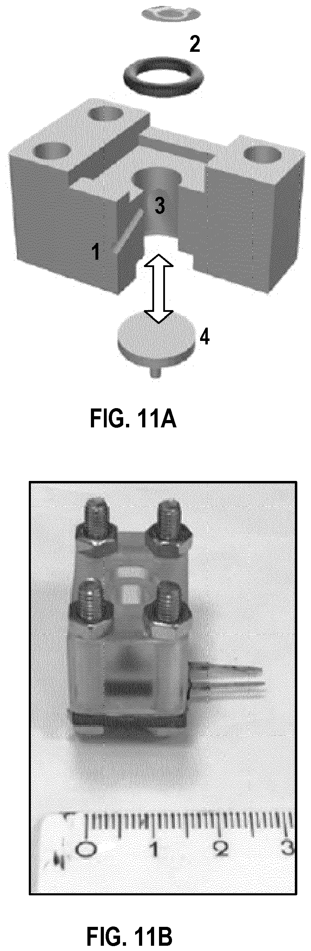

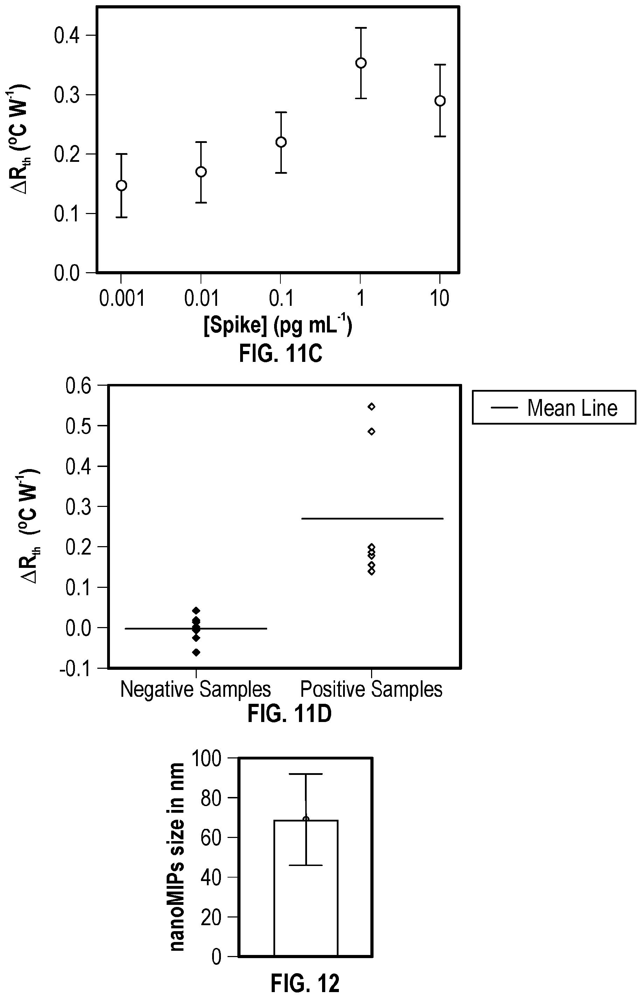

Figure 11 shows binding between nanoMIPs imprinted against peptide 1 and SARS-CoV-2 from clinical samples. Figure 11a is a schematic illustration of a 3D-printed prototype addition cell: 1) thermocouple inlet, 2) functionalized SPE, 3) open-bottomed reservoir to facilitate the manual addition of liquid, and 4) removable lid to reduce experimental noise. Figure 11b is a photograph of the addition cell shown in Figure 11a. Figure 11c shows a typical dose-response curve obtained when using the prototype addition cell for the thermal detection of the SARS-CoV-2 spike protein (1 fg mL$^{-1}$ - 10 pg mL$^{-1}$) in phosphate-buffered saline (PBS). Figure 11d shows the thermal response of the nanoMIP sensor to clinical samples from COVID-positive and negative patients (n = 7);

Figure 12 shows the size of nanoMIPs measured using NanoSight particle analyzer;

Figure 13 shows nanoMIP - LSPR sensor mediated detection of SARS-CoV-2 spike proteins from SARS-CoV-2 Alpha, Beta and Gamma variants in PBS. Figure 13a shows an LSPR spectrum in wavelength vs absorbance plot for concentrations ranging from 10 aM to 100 nM of the Alpha variant of SARS CoV-2. Figure 13b shows the change in LSPR wavelength of the Ag-MIPs complex upon varying the concentration of SARS-CoV-2 Alpha, Beta and Gamma variants from 10 aM to 100 nM;

Figure 14 shows nanoMIP - LSPR mediated detection of SARS-CoV-2 spike proteins from SARS-CoV-2 Alpha, Beta and Gamma variants in serum. Figure 14a shows an LSPR spectrum in wavelength vs absorbance plot for concentrations ranging from 100 fM to 100 nM of the Alpha variant of SARS-CoV-2. Figure 14b shows the change in LSPR wavelength of the Ag-MIPs complex upon varying the concentration of SARS-CoV-2 Alpha, Beta and Gamma variants from 100 fM to 100 nM; and

Figure 15 shows two superimposed surface plasmon resonance sensorgrams showing the binding kinetics for nanoMIPs imprinted against peptide 1 and either the original SARS-CoV-2 S (spike) recombinant protein and or that from the Omicron variant.

Examples, or parts thereof, which relate to molecularly imprinted polymers comprising at least one recognition site that is complementary to a template molecule consisting of an amino acid sequence corresponding to a subsequence of the receptor binding domain of SARS-CoV-2 wherein the amino acid sequence does not comprise the sequence NSNNLDSKVGG are not part of the claimed invention and are present for comparative purposes only.

## Example 1: Synthesis of NanoMIPs to SARS-CoV-2

## Selection of three peptides from SARS-CoV-2 suitable for imprinting

Supplementary Figure 5 of Wrapp *et al* 2020[1] presents a sequence alignment of the spike protein from SARS-CoV-2 and two other coronavirus strains, SARS-CoV and RaTG13. The RBD is highlighted. Three peptide sequences (peptide 1: NSNNLDSKVGG, peptide 2: STEIYQAGSTPC and peptide 3: CYFPLQSYGFQP) from the RBD of SARS-CoV-2 were selected by the present inventors as potential candidates for imprinting based on the lack of conservation with the respective sequences from SARS-CoV and RaTG13.

## Solid-Phase synthesis of NanoMIPs to SARS-CoV-2

Peptides 1, 2 and 3 were immobilised onto silanised glass beads prior to nanoMIP synthesis.

On the basis that a terminal cysteine was required for peptide immobilisation, when peptides 1, 2 and 3 were ordered from Ontores, China, a cysteine residue was added to the N terminus of peptide 1. A further glycine residue was also added between the cysteine and the N terminal asparagine, functioning as a spacer. Thus, the peptides sourced from Ontores for use in imprinting were:

Peptide 1: CGNSNNLDSKVGG
Peptide 2: STEIYQAGSTPC
Peptide 3: CYFPLQSYGFQP.

Peptide immobilisation was carried out as follows: glass beads (approximately 100 $\mu$M in diameter, sourced from Microbeads AG) were activated by boiling in 4M NaOH for 10 minutes. The glass beads were then washed with firstly deionised water and then acetone and then dried at 80°C for 2 hours. The glass beads were then incubated in toluene with 2 % v/v (3-aminopropyl)trimethoxysilane (APTMS) for 3 hours, washed with acetone and placed in PBS, pH 7.4 with 0.2mg/ml n-succinimidyl iodoacetate (SIA) for two hours before being washed with acetonitrile. The templates (Peptides 1, 2 or 3) were then immobilised on the surface of the glass beads by incubation in a solution of PBS, pH 7.4 with 0.4 mg/ml tris(2-carboxyethyl) phosphine hydrochloride (TCEP) and 0.1mg/ml of peptide for a minimum of 4 hours. Excess template was removed by washing with water and methanol.

The peptide coated glass beads were used for the synthesis of imprinted nanoMIPs as follows: monomer solutions were prepared, sonicated for 10 minutes and purged with nitrogen for 5 minutes. One example of a monomer solution that was prepared includes 3mg N-Fluoresceinyl acrylamide, 24.2mg Acrylamide, 31.7$\mu$L Tert-Butyl acrylate (TBAc), 36mg 3-O-Acryloyl-1,2:5,6-bis-O-isopropylidene-D-glucofuranose, 13.8$\mu$L 2,2,2-Trifluoroethyl methacrylate (CF3), 3.9mg N-(3-Aminopropyl) methacrylamide hydrochloride (APMA), 1.3mg Squareamide monomer 7 (SQ7) and 9mg N,N'-Methylenebis(acrylamide) (BIS)). 100ml of the monomer solution was then put into a 250ml Duran flask containing 60g of the peptide coated glass beads. Polymerisation was then initiated by adding 0.5ml of 60 mg/ml ammonium persulphate (APS) with 60$\mu$L/ml of N,N,N',N'-tetramethylethylenediamine (TEMED) and carried out at room temperature for 1 hour. The contents of the flasks were then poured into Solid Phase Extraction (SPE) cartridges fitted with 20$\mu$M porosity frits in order to separate the glass beads with attached nanoparticles from the other components. 10 washing steps, each with approximately 25ml of room temperature water, were then performed to remove low affinity material. The residue from each of these washing steps was discarded. High affinity nanoMIPs were then detached from the glass beads by performing 9 washing steps, each with 10ml of 60°C ethanol. The residue from each of these washes, containing the nanoMIPs, was collected. The nanoMIPs were then concentrated down to approximately 10ml and solvent exchanged to distilled water. Imprinted nanoMIPs had an average diameter of 59nm, as calculated by NanoSight NS300.

Example 2: Surface Plasmon Resonance (SPR) Analysis of NanoMIPs to SARS-CoV-2 peptides

A Biacore 3000 instrument was employed in this study.

Surface activation and nanoMIP immobilisation.

NanoMIP-SPR sensors were prepared by covalently immobilising nanoMIPs (from Example 1) on 4% mercaptoundecanoic acid (MUDA) chips (prepared using Biacore SIA Kit Au surfaces, functionalised with a self-assembled monolayer of 4% MUDA in ethanol).

In more detail, water was utilized to prime the instrument at a constant flow rate of $5\mu L$/minute. Water was utilized as the running buffer throughout the chip preparation at a constant flow rate of $30\mu l$/minute. Water was run until a stable baseline was achieved. A mixture of N-hydroxysuccinimide (NHS) and 1-ethyl-3-(3-dimethylaminopropyl)-carbodiimide (EDC) (2 mg EDC + 3 mg NHS in 400 uL of water) was injected (8 minutes at a flow rate of $8\mu L$/minute) across the sensor surface to activate the surface (the mixture converts the carboxylic terminal groups on the chip surface into N-Hydroxy-succinimide esters). NanoMIPs (5nM) were then injected (8-10 minutes at a flow rate of $8\mu L$/minute) into all four flow cells on the chip. Finally, unreacted NHS esters were hydrolysed by injecting carbonate buffer (pH 9.2) (30 minutes at a flow rate of between 5 and $30\mu L$/minute).

Detection of interaction between nanoMIPs and SARS-CoV-2 peptides

Prior to testing in SPR, each of peptides 1, 2 and 3 were conjugated to Bovine Serum Albumin (BSA).
Once a stable baseline was obtained having switched running buffer from water to PBS, 7 dilutions of BSA or BSA-peptide conjugates (2.33mg/ml, 0.777mg/ml, 259ug/ml, 86ug/ml, 28.8ug/ml, 9.6ug/ml, 3.2ug/ml in PBS) were injected (5 minutes association phase, 4 minutes dissociation phase, $28\mu l$/minute flow rate) as follows: BSA alone was injected into flow cell 1, BSA-peptide 1 into flow cell 2 and BSA-peptide 3 into flow cell 3.
Good selectivity was observed for nanoMIPs imprinted against peptide 3, for which the software calculated a $K_D$ of 160 nM (data not shown).

**Example 3: Surface Plasmon Resonance Analysis of NanoMIPs to SARS-CoV-2 S protein**

A Biacore 3000 instrument was employed in this study.

Surface activation and SARS-CoV-2 S protein immobilisation.

SARS-CoV-2 S protein-SPR sensors were prepared by covalently immobilising recombinant SARS-CoV-2 S protein (recombinant full-length SARS-CoV-2 spike glycoprotein sourced from The Native Antigen Company) on 4% mercaptoundecanoic acid (MUDA) chips (prepared using Biacore SIA Kit Au surfaces, functionalised with a self-assembled monolayer of 4% MUDA in ethanol).
In more detail, water was utilized to prime the instrument at a constant flow rate of 5 $\mu l$/minute. Water was also utilized as the running buffer throughout the chip preparation at a constant flow rate of $30\mu l$/minute. Water was run until a stable baseline was achieved. A mixture of N-hydroxysuccinimide (NHS) and 1-ethyl-3-(3-dimethylaminopropyl)-carbodiimide (EDC) (2 mg EDC + 3 mg NHS in 400 uL of water) was injected (8 minutes at a flow rate of $8\mu l$/minute) across the sensor surface to activate the surface (the mixture converts the carboxylic terminal groups on the chip surface into N-Hydroxy-succinimide esters). Control (1% BSA *(w/v)* and 0.5% Pluronic F127 *(w/v))* and two different concentrations of recombinant SARS-CoV-2 S protein (60nM and 600nM) were then injected (6 minutes 15 seconds at a flow rate of $8\mu l$/minute) into flow cells 1, 2 and 3 respectively on the chip. Finally, unreacted NHS esters were hydrolysed by injecting carbonate buffer (pH 9.2) (1 minute at a flow rate of $30\mu l$/minute).

Detection of interaction between SARS-CoV-2 S protein and nanoMIPs

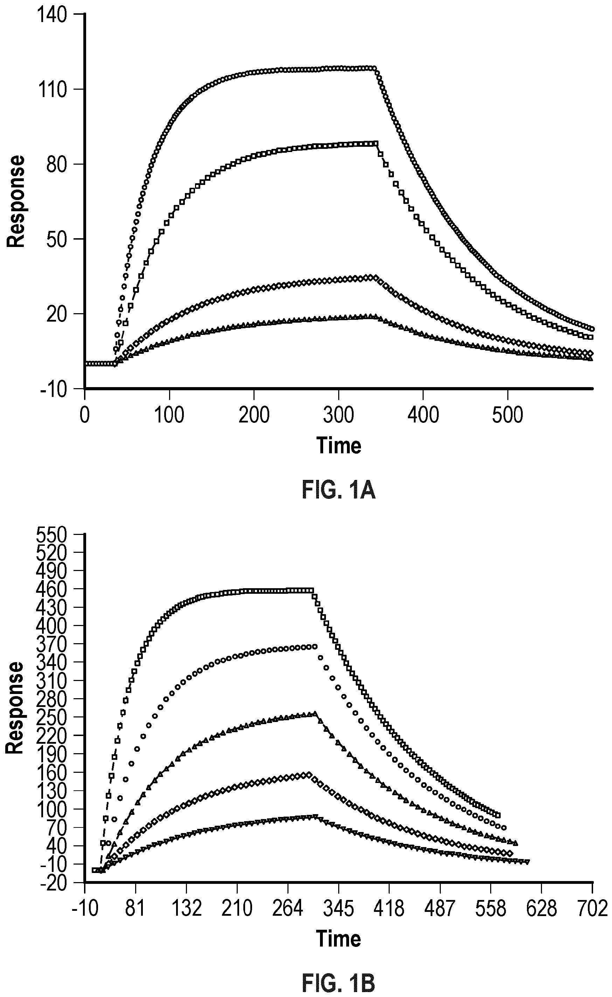

Once a stable baseline was obtained having switched running buffer from water to PBS, 30nM (and 1:1 serial dilutions thereof) nanoMIPs imprinted against peptide 1 were then injected (5 minutes association phase, 3 minutes dissociation phase, $28\mu l$/minute flow rate).
The software calculated a $K_D$ of 5nM (Figure 1a) and 15nM (Figure 1b).

**Example 4: Conjugation of NanoMIPs to SARS-CoV-2 to CPN**

NanoMIPs imprinted against peptides 1 and 3 were coupled to Conjugated Polymer Nanoparticles (CPNs), highly fluorescent nanoparticles containing semiconductor Light Emitting Polymer cores encapsulated within a water friendly capping agent, at Stream Bio. The CPNs used were CPN510 and CPN610 which have a diameter of 70-80nm as measured by NanoSight 3000.

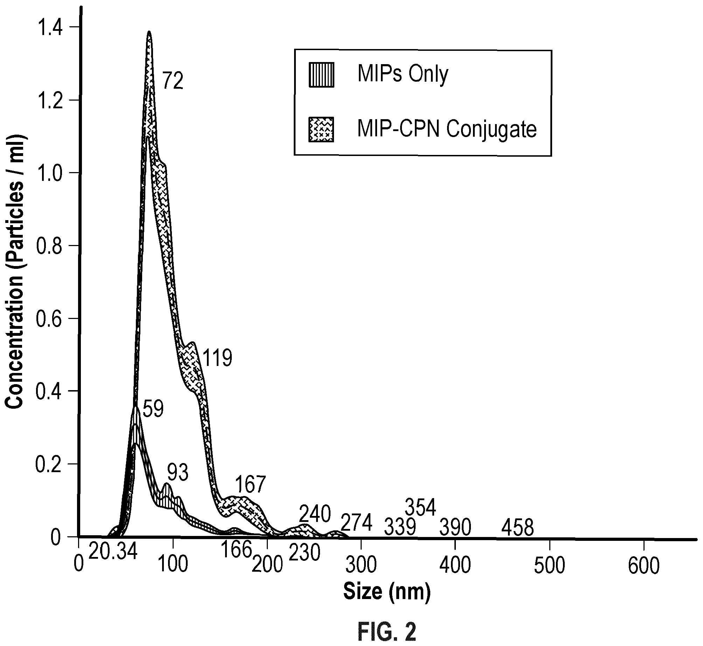

Figure 2 shows the successful conjugation of a nanoMIP imprinted against peptide 1 to CPN510. Note that the original unconjugated nanoMIPs were predominantly 59nm; however, when conjugated to CPN510, a significant shift in particle size is seen - see the peak at 119nm.

**Example 5: Dot-blot analysis of binding between NanoMIPs and SARS-CoV-2 S protein.**

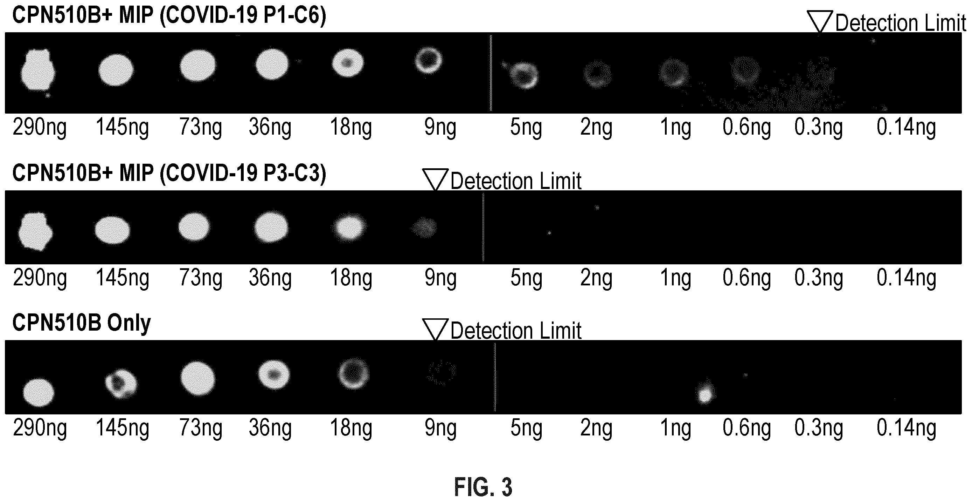

Different concentrations (290ng, 145ng, 73ng, 36ng, 18ng, 9ng, 5ng, 2ng, 1ng, 0.6ng, 0.3ng and 0.14ng) of recombinant SARS-CoV-2 S protein (sourced from The Native Antigen Company) were blotted onto nitrocellulose membranes before allowing the membranes to dry. The membranes were then blocked by soaking in 5% BSA in PBS-T (0.05% Tween-20 in PBS) for 30-60 minutes at room temperature. Post blocking, the membranes were incubated with (1) NanoMIP-CPN510 (imprinted against peptide 1), (2) NanoMIP-CPN510 (imprinted against peptide 3) or (3) CPN510 only (control) dissolved in 5% BSA in PBS-T for 30-60 minutes at room temperature. The membranes were then washed three times, for 5 minutes each time, with PBS-T. Fluorescence was then observed under UV. As shown in Figure 3, the detection limit using NanoMIP-CPN510 (imprinted against peptide 1) was 0.3ng, much lower than that achieved using CPN510 only (control) indicating that nanoMIPs imprinted against at least peptide 1 specifically interact with SARS-CoV-2 S protein.

**Example 6: Dot-blot analysis of binding between NanoMIPs and SARS-CoV-2 virus.**

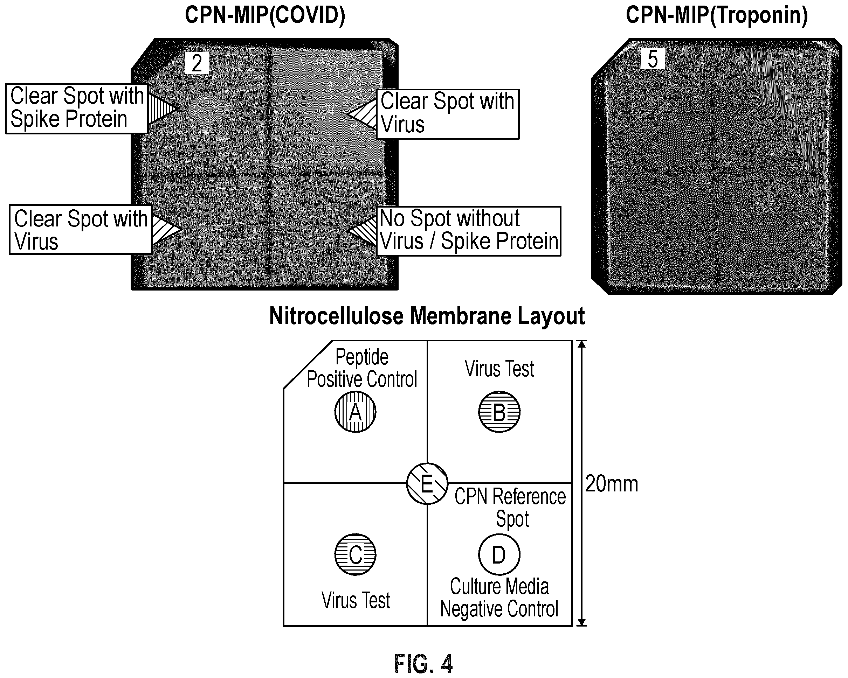

SARS-CoV-2 ($2 \times 10^4$ pfu) was blotted onto two nitrocellulose membranes. Also blotted on to each of the nitrocellulose membranes was 75ng of recombinant SARS-CoV-2 S protein (sourced from The Native Antigen Company) as a positive control and culture media as a negative control. The membranes were then left to dry. The membranes were then blocked by soaking in 5% BSA in PBS-T (0.05% Tween-20 in PBS) for 30-60 minutes at room temperature. Post blocking, the membranes were incubated with NanoMIP-CPN510 (imprinted against peptide 1) or (2) NanoMIP-CPN510 (imprinted against troponin) (negative control) dissolved in 5% BSA in PBS-T for 30-60 minutes at room temperature. The membranes were then washed three times, for 5 minutes each time, with PBS-T. Fluorescence was then observed under UV. As shown in Figure 4, no fluorescence was observed in the area of the nitrocellulose membrane blotted with culture media or when the membrane was incubated with NanoMIP-CPN510 (imprinted against troponin) (negative control). In contrast, fluorescence was observed when the nitrocellulose membrane was incubated with NanoMIP-CPN510 (imprinted against peptide 1) both with respect to SARS-CoV-2 and also the SARS-CoV-2 S protein positive control. This indicates that nanoMIPs imprinted against at least peptide 1 specifically interact with SARS-CoV-2.

**Example 7: Dot-blot analysis comparing binding between (1) NanoMIPs and four different human coronaviruses (SARS-CoV-2, HCoV-OC43, HCoV-229E and HCoV-HKU1) and (2) antibodies and four different human coronaviruses (SARS-CoV-2, HcoV-OC43, HCoV-229E and HCoV-HKU1).**

0.5 μL of SARS-CoV-2 Spike Glycoprotein (S1) (The Native Antigen Company), Human Coronavirus OC43 Spike Glycoprotein (S1) (The Native Antigen Company), Human Coronavirus 229E Spike Glycoprotein (S1) (The Native Antigen Company) and Human Coronavirus HKU1 Spike Glycoprotein (S1) (The Native Antigen Company) (0.6 mg/mL) were blotted onto nitrocellulose membranes in triplicate (as shown in the schematic in Figure 5) before allowing the membranes to dry. The membranes were then blocked by soaking in 2 mL BSA (1% in PBS) for 1 hour. Post blocking, the membranes were incubated with (1) NanoMIP - CF770 (imprinted against peptide 1) and (2) three different monoclonal antibodies specific to SARS-CoV-2 Spike (RBD), Mab RBD1106-CF770, Mab RBD107-CF770 and Mab RBD5305-CF770 (all prepared in-house and diluted in 0.01% BSA) for 30 minutes. The membranes were then washed with 2mL PBST for 5 minutes. Fluorescence was then observed under UV. As shown in Figure 5, the nanoMIPs selectively bound the SARS-CoV-2 Spike Glycoprotein (S1) and did not bind to any of Human Coronavirus OC43 Spike Glycoprotein (S1), Human Coronavirus 229E Spike Glycoprotein (S1) or Human Coronavirus HKU1 Spike Glycoprotein (S1). Furthermore, the nanoMIPs were shown to have comparable selectivity and binding to each of the antibodies tested.

**Example 8: Analysis of binding between NanoMIPs and SARS-CoV-2 S protein RBD using thermal detection methods.**

Preparation of nanoMIP-functionalized Screen-Printed Electrodes (SPEs) for use in thermal detection methods.

A solution of 4-ABA (2 mM) and sodium nitrite (2 mM) in aqueous HCl (0.5 M) was prepared and gently mixed on an orbital shaker for 10 minutes. Screen-Printed Electrodes (SPEs) were submerged into the solution and cyclic voltammetry was performed from +0.2 V to -0.6 V at 100 mV s$^{-1}$ using an Ag/AgCl reference electrode (Alvatek Ltd., Romsey, UK). The obtained electrode, denoted SPE/4-ABA, was thoroughly rinsed with deionised water to remove any unbound 4-ABA and dried with nitrogen. The carboxyl group was then activated through incubation with a solution of 1-ethyl-3-(3-dimethylaminopropyl)-carbodiimide (EDC) (100 mM) and N-hydroxysuccinimide (NHS) (20 mM) in PBS buffer (pH = 5). Dropcasting was utilised to deposit 8 $\mu$L of the EDC/NHS solution onto the working electrode of the SPEs. After 1 hour, the electrodes were rinsed with deionised water and dried to obtain the SPE/4-ABA/EDC+NHS electrodes. Following this, 8 $\mu$L of a given nanoMIP solution (NanoMIP imprinted against peptide 1 'P1C6' or NanoMIP imprinted against peptide 3 'P3C6') was deposited onto the working electrodes following a gentle vortex. After 3 hours, the SPE/4-ABA/EDC+NHS/-nanoMIP electrodes were rinsed in deionised water and dried with a gentle stream of nitrogen before being stored in PBS at 4°C until use.

Detection of binding between **NanoMIPs** and SARS-CoV-2 S protein RBD using nanoMIP-functionalised Screen-Printed Electrodes (SPEs).

3D-printed flow cells were used to facilitate measurements using the nanoMIP-functionalised SPEs described above. A SPE was mounted into the cell and a thermocouple measured the temperature in the liquid ($T_2$). For each measurement, a freshly prepared nanoMIP-functionalised SPE was used. The flow cell was connected to a heat-transfer device as described by van Grinsven *et al.* The device was steered with LabView software that actively controls the temperature of the heat sink (copper block, $T_1$), which was set to 37.00 $\pm$ 0.02 °C to mimic *in-vivo* conditions. A proportional-integral-derivative (PID) controller attached to a power resistor (22 $\Omega$) regulated the feedback on the signal as described in Geerets *et al.* The PID parameters were fixed for all experiments at optimised values of P=1, I=10, and D=0.2.

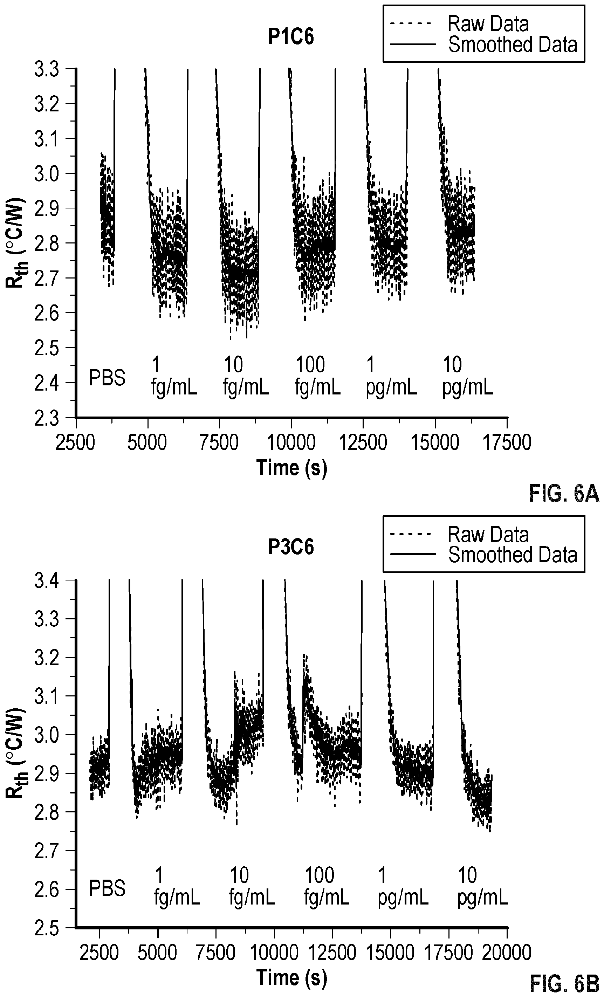

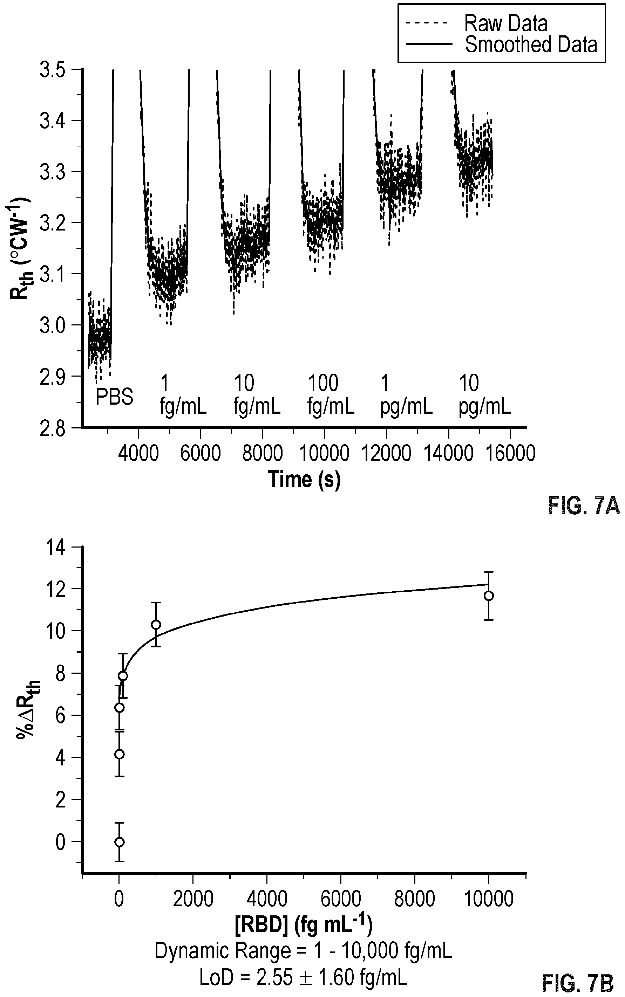

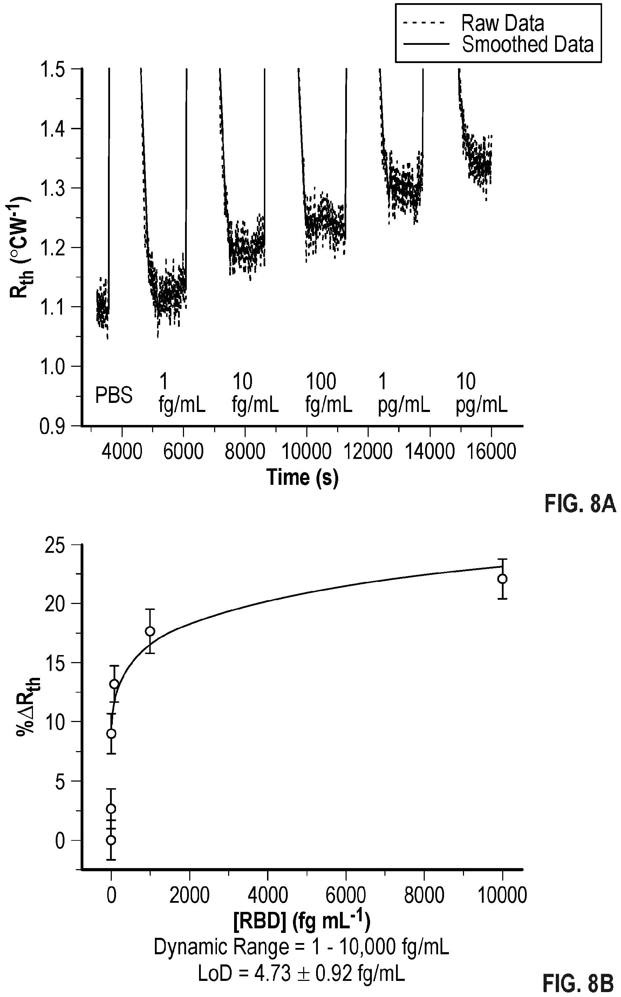

In all measurements, the flow cell was filled with PBS and left for 30 minutes to ensure stabilisation of the baseline temperature signal before a first injection of PBS was added to act as the blank measurement. Solutions (3 mL) of increasing concentrations of each target biomarker (SARS-CoV-2 S protein RBD or SARS-CoV-2 ORF8 (as a negative control)) (0-10 pg mL$^{-1}$) were prepared in PBS prior to experiments and stored at 4°C until required. Each biomarker injection was performed at 250 $\mu$L min$^{-1}$ for 12 minutes using a LSP02-1B dual channel syringe (Longer Precision Pump Co., Hebei, China) pump. Following each biomarker injection, the system was allowed to stabilise for 30 minutes prior to the next injection. The thermal resistance ($R_{th}$) was determined throughout the experiments by dividing the temperature gradient ($T_1$-$T_2$) over the power required to keep the heat sink at 37.00°C. The $R_{th}$ and standard deviation (SD) were calculated using the average of 600 data points from the baseline signal of each concentration and the initial PBS injection, respectively (Figures 6, 7a, and 8a). This data was used to construct dose-response curves, from which the limit of detection (LoD) was calculated using the three-sigma method in the linear range of the sensor (Figures 7b and 8b). The error bars in the graphs relate to the SD values. Figure 6 shows that the thermal response to SARS-CoV-2 ORF8 is very limited whereas Figures 7 and 8 show that a good response was observed with SARS-CoV-2 S protein RBD for nanoMIPs imprinted against both peptides 1 and 3.

## Example 9: Analysis of nanoMIP robustness

The capability of MIPs imprinted against peptide 1 to withstand extremes of temperature and pH was investigated.

Temperature

Atomic force microscopy (AFM) was utilized to examine how increasing temperature impacted the morphology of adsorbed nanoMIPs by imaging the same nanoMIPs at room temperature, 37°C, and 50°C.

AFM measurements were performed on a JPK Nanowizard 4 XP Bioscience (Bruker, Nano GmbH, Berlin, Germany). Measurements in air were carried out in tapping mode using PPP-NCL-W probes (Nanosensors, Neuchatel, Switzerland) with a cantilever length of ~225 $\mu$m and spring constant of ~48 N m$^{-1}$. Measurements in liquid were performed in Quantitative Imaging (QI) mode using MLCT-E probes (Bruker, Ca, USA) with a cantilever length of ~140 $\mu$m and spring constant of ~0.1 N m$^{-1}$. Au-coated Si chips were used as substrates (Si-Mat, Kaufering, Germany). Prior to drop-casting, the chips were cleaned by immersion for 5 min in a 5:1:1 mixture of milli-Q water, ammonia, and hydrogen peroxide heated at 75 °C. The chips were then rinsed in milli-Q water and dried with nitrogen. NanoMIP solutions were diluted in milli-Q water to ~2.54 $\mu$g mL$^{-1}$, drop-cast (20 $\mu$L) onto the Au-coated surfaces, and allowed to dry in ambient conditions for a minimum of 4 h in a Petri dish. A high temperature heating stage (HTHS, JPK BioAFM - resolution of 0.1 °C) was used as a temperature controller to facilitate imaging at 37°C and 50°C.

Figure 9a and 9b which show typical AFM images and a corresponding cross-sectional profile plot respectively show that the nanoMIP morphology was unaffected by increasing temperature.

NanoMIP volume was calculated using Gwyddion. Tracking numerous droplets revealed minimal changes in mean nanoMIP volume (~6% decrease) from room temperature to 50 °C.

Thus, nanoMIP morphology remains consistent across relatively large temperature ranges.

SPR binding analysis, using a Biacore 3000 instrument, was also performed against the SARS-CoV-2 spike protein using nanoMIPs that had experienced autoclaving for 15 minutes with a maximum temperature of 121°C.

In more detail, two nanoMIP-SPR sensors were prepared by covalently immobilising nanoMIPs (from Example 1), either as directly prepared or after autoclave at 121°C for approximately 15 minutes, on 4% mercaptoundecanoic acid (MUDA) chips (prepared using Biacore SIA Kit Au surfaces, functionalised with a self-assembled monolayer of 4% MUDA in ethanol) in line with the process directly below.

Water was utilized to prime the instrument at a constant flow rate of $5\mu L$/minute. Water was utilized as the running buffer throughout the chip preparation at a constant flow rate of $30\mu l$/minute. Water was run until a stable baseline was achieved. A mixture of N-hydroxysuccinimide (NHS) and 1-ethyl-3-(3-dimethylaminopropyl)-carbodiimide (EDC) (2 mg EDC + 3 mg NHS in 400 uL of water) was injected (8 minutes at a flow rate of $8\mu L$/minute) across the sensor surface to activate the surface (the mixture converts the carboxylic terminal groups on the chip surface into N-Hydroxy-succinimide esters). NanoMIPs (5nM) were then injected (8-10 minutes at a flow rate of $8\mu L$/minute) into all four flow cells on the chip. Finally, unreacted NHS esters were hydrolysed by injecting carbonate buffer (pH 9.2) (30 minutes at a flow rate of between 5 and $30\mu L$/minute).

Once a stable baseline was obtained having switched running buffer from water to PBS, 5 dilutions (30, 15, 7.5, 3.75 and 1.88mM in PBS) of SARS-CoV-2 S protein (recombinant full-length SARS-CoV-2 spike glycoprotein sourced from The Native Antigen Company) were injected (circa 5 minutes association phase, at least 2 minutes dissociation phase, $28\mu l$/minute flow rate) with suitable controls in control channels. The results from the two chips were then superimposed, and the software calculated very similar KD values for the nanoMIPs before and after autoclaving (7 and 3 nM, respectively), demonstrating that there is no impact on binding affinity after exposure to high temperatures. This is highly advantageous for nanoMIP shelf-life as autoclaving (sterilization) facilitates their long-term storage in water without bacterial degradation. In contrast, antibodies experience significant deterioration in affinity at temperatures above 37 °C.

pH

AFM was also used to assess the ability of nanoMIPs to withstand extremes of pH by measuring the volume of adsorbed nanoMIPs (n = 120) in liquid at various pH levels. More specifically, QI measurements were performed at room temperature (23 $\pm$ 1 °C) in liquid at different pH levels (5.5-8.5) within the operating conditions of the AFM. Initial measurements were carried out in pure milli-Q water (pH 5.5) and the pH was subsequently increased with the addition of ammonia.

As above, nanoMIP volume was calculated using Gwyddion which revealed negligible changes to the mean nanoMIP volume from pH 5.5 to 8.5 (~3% decrease), highlighting that adsorbed nanoMIP morphology was consistent across a broad pH range (see Figure 9c).

Additionally, the thermal response of the developed nanoMIP tests (see Example 10) to a clinical reference liquid (universal transport medium, UTM) and Diet Coca-Cola (pH = 3.5) were compared. The results demonstrated that there was no statistically significant difference between the thermal response of the two liquids (see Figure 9d). In contrast, commercial antibody-based rapid antigen tests are highly impacted by changes to pH and false positive results are produced when acidic soft drinks (Diet Coca-Cola) are used as the test liquid (see Figure 9e).

**Example 10: Comparison of binding between NanoMIPs and SARS-CoV-2 antigens and SARS-CoV-2 antibodies and SARS-CoV-2 antigens using thermal detection methods.**

NanoMIP-functionalized Screen-Printed Electrodes (SPEs) (using nanoMIPs imprinted against peptide 1) were made in line with the procedure in Example 8.

SARS-CoV-2 antibody-functionalized SPEs were also made in line with the procedure in Example 8.

Detection of binding between NanoMIPs or SARS-CoV-2 antibodies and SARS-CoV-2 antigens using nanoMIP- or SARS-CoV-2 antibody-functionalised Screen-Printed Electrodes (SPEs).

SARS-CoV-2 antigens (either recombinant full-length SARS-CoV-2 spike glycoprotein (alpha variant) sourced from The Native Antigen Company, recombinant SARS-CoV-2 spike receptor binding domain (RBD) (delta variant) sourced from Abbexa, Cambridge, UK or SARS-CoV-2 RBD from the Medical Research Council Protein Phosphorylation and Ubiquitylation Unit (Dundee, UK)) were thermally detected by mounting nanoMIP-functionalized SPEs into 3D-printed resin flow cells to create an interface between the heat sink and liquid reservoir. Two thermocouples measured the heat sink ($T_1$) and liquid reservoir ($T_2$) temperatures every second, and the thermal resistance ($R_{th}$) was obtained by dividing the temperature gradient ($T_1$-$T_2$) over the power required to maintain the heat sink at 37.00 $\pm$ 0.02 °C. As the target attached to the nanoMIPs, heat transfer at the solid-liquid interface was reduced (larger temperature gradient), which led to a measurable increase in the $R_{th}$.

For all experiments, the thermal measurement device was controlled using LabView software and a proportional-integral-derivative (PID) controller attached to a power resistor (22 $\Omega$) regulated the feedback on the signal. The PID parameters were optimized to reduce noise and were set at P = 1, $I$ = 13, and D = 0.2.

The liquid reservoir was filled with PBS and left for 30 min to ensure stabilization of the baseline $R_{th}$ signal. Subsequently, five spiked PBS solutions (3 mL) with increasing concentrations of the SARS-CoV-2 antigen (1 fg mL$^{-1}$ - 10 pg mL$^{-1}$) were injected into the flow cell at a rate of 250 $\mu$L min$^{-1}$ for 12 min using an automated syringe pump (LSP02-1B, Longer Precision Pump Co., Hebei, China). The system was allowed to stabilize for 30 min prior to each subsequent injection. The experiments produced raw thermal data plots which displayed a step-wise increase in $R_{th}$ where each stabilized plateau represents the injection of an increasingly concentrated spiked solution (see Figure 10a). Dose-response curves (Figures 10b-d) were composed from the thermal plots by taking the mean and standard deviation (SD) of the stabilized plateau from each concentration injection. Limit of detection (LoD) values were calculated from the dose-response curves using the three-sigma method (3 $\times$ reference SD) in the linear range.

SARS-CoV-2 antibody-functionalized SPEs were used to facilitate a direct comparison between the sensing performance of the nanoMIPs and those of antibody receptors. The thermal detection results (see Figure 10b) revealed that the response to the SARS-CoV-2 spike protein (alpha variant) was very similar for the nanoMIP (LoD = 9.9 $\pm$ 2.5 fg mL$^{-1}$) and antibody (LoD = 8.9 $\pm$ 4.1 fg mL$^{-1}$) based sensors highlighting that the specificity of the nanoMIPs against the spike protein is comparable with antibodies used in commercial tests.

The ability of the nanoMIP receptors to detect virus mutations was also examined by measuring their specificity against the SARS-CoV-2 spike protein (delta variant). Results show (Figure 10b) that the nanoMIP sensor was effective in detecting the delta variant as the resulting LoD (6.1 $\pm$ 2.9 fg mL$^{-1}$) was very similar to the value obtained for the alpha variant. This demonstrates that the nanoMIP sensor can detect equally low amounts of both the alpha and delta variant spike protein, which is highly promising for potential commercial applications where there is an ongoing concern regarding the reduction in test efficacy due to the presence of new variants.

The versatility of nanoMIP receptors was also examined by measuring their thermal response against the SARS-CoV-2 RBD (see Figure 10c). Antibody receptors were also tested for the purpose of direct comparison. The LoD value for the SARS-CoV-2 RBD was ~20 times smaller for the nanoMIP sensor (3.9 $\pm$ 1.0 fg mL$^{-1}$) compared to the antibody sensor (85.5 $\pm$ 15.0 fg mL$^{-1}$). Consequently, this demonstrates that the nanoMIPs possessed greater versatility than antibodies as they had a comparable LoD for the full-length spike protein but a significantly lower LoD for the RBD.

The selectivity of the nanoMIP sensor was also comprehensively examined using three negative controls which are common interferents in clinical samples: open reading frame 8 (ORF8), interleukin-6 (IL-6), and human serum albumin (HSA). A high degree of binding occurred between the SARS-CoV-2 antigens and nanoMIPs, which led to large $\Delta R_{th}$ values at the highest concentration (10 pg mL$^{-1}$) for the spike protein (0.23 °C W$^{-1}$) and RBD (0.35 °C W$^{-1}$). In contrast, minimal binding occurred with the negative controls, which led to significantly lower $\Delta R_{th}$ values for ORF8 (0.00 °C W$^{-1}$), IL-6 (0.06 °C W$^{-1}$), and HSA (0.05 °C W$^{-1}$). The results demonstrated that the thermal response of the nanoMIP sensor was considerably greater for SARS-CoV-2 antigens compared to the negative controls, which highlighted the excellent selectivity of the nanoMIP sensor. An additional control experiment was also performed using non-imprinted polymers (NIPs) immobilized to SPEs (NIP functionalised SPEs made in line with the procedure in Example 8). NIPs were prepared using the same synthesis protocol as nanoMIPs except they were not exposed to a target epitope during polymerization, and therefore had no specific cavities to facilitate target binding. The thermal response of a nanoMIP-functionalized SPE to the spike protein was ~6 times larger compared to the NIP-functionalized SPE (0.04 °C W$^{-1}$) showing that specific binding occurred between the spike protein and nanoMIP cavities.

The LoD values of the nanoMIP sensor, a commercial rapid antigen test, and numerous recently developed antigen tests from the literature[4-15] are presented in Figure 10e. Table 1 below provides further details of the experimental setup. The LoD value for the nanoMIP sensor is ~6000 times lower than the commercial rapid antigen test (20 pg mL$^{-1}$). This considerably lower LoD highlights that nanoMIP receptors could be a valuable tool in producing rapid antigen tests with adequate sensitives for effective population screening. Additionally, the LoD of the nanoMIP sensor is among the lowest value of those from recently developed antigen tests in the literature. This demonstrates that thermal detection using nanoMIPs can compete with the best performing antigen tests from recent literature, whilst possessing the added benefit of being able to withstand extremes of temperature and pH.

<u>Table 1</u>

| Biosensor[a] | Method[b] | Analyte[c] | Limit of detection | Label in Fig. 10e | Ref |
|---|---|---|---|---|---|
| NanoMIP-based thermal sensor | NanoMIPs immobilized onto SPEs. HTM used for detection. | RBD and spike protein | RBD: 3.9 fg mL$^{-1}$ S protein: 9.9 fg mL$^{-1}$ | 1 | Current work |
| WANTAI SARS-CoV-2 Ag Rapid Test LFA | Lateral flow immunochromatography combined with double antibody sandwich method. | Nucleocapsid protein | 20 pg mL$^{-1}$ | 2 | 4 |
| Half-strip LFA | Test strip with immobilized antibodies and optical read out. | Nucleocapsid protein | 0.65 ng mL$^{-1}$ | 3 | 5 |
| Fluorescent microsphere LFA biosensor | Test strip combining fluorescent microsphere labelling and immunochromatography technology. | Nucleocapsid protein | 100 ng mL$^{-1}$ | 4 | 6 |
| Nanozyme CL paper test | Nanozyme and enzymatic CL immunoassay combined with a lateral flow strip. | Spike protein | 100 pg mL$^{-1}$ | 5 | 7 |
| Electrochemical immunosensor with Cu$_2$O nanocube coating | Spike antibodies immobilized onto SPEs modified with Cu$_2$O nanocubes. CV and EIS used for detection. | Spike protein | 0.04 fg mL$^{-1}$ | 6 | 8 |
| MIP-based electrochemical sensor | Disposable Au-TFE chip modified with ncovNP-MIPs. DPV used for detection. | Nucleocapsid protein | 0.7 pg mL$^{-1}$ | 7 | 9 |
| FET-based biosensor | Electrical measurements using graphene-based FET functionalized with spike antibodies. | Spike protein | 1 fg mL$^{-1}$ | 8 | 10 |
| Colorimetric assay using AuNPs | AuNPs capped with thiol-modified ASOs specific for nucleocapsid protein. SPR used for detection. Addition of RNase H produces a precipitate for visual detection. | Nucleocapsid protein | 180 ng mL$^{-1}$ | 9 | 11 |
| Cell-based biosensor | Mammalian Vero cells engineered by electroinsertion of spike antibodies. Change in bioelectric properties measured with a cell-biosensor set up using the principles of BERA. | Spike protein | 1 fg mL$^{-1}$ | 10 | 12 |
| SERS-based biosensor | AuNPs immobilized onto Si wafer to form SERS-active substrate with spike antibodies attached. Raman reporter-labelled immuno-AgNPs used as SERS nanotags for detection. | Spike protein | 6 fg mL$^{-1}$ | 11 | 13 |
| ePAD | Spike antibody immobilized onto ePAD surface. SWV used for detection. | Spike protein | 110 pg mL$^{-1}$ | 12 | 14 |
| Magnetic bead-based electrochemical immunosensor | Magnetic beads used as support for immunological chain and combined with a carbon black-based SPE. DPV used for detection. | Spike and nucleocapsid proteins | S protein: 19 ng mL$^{-1}$ N protein: 8 ng mL$^{-1}$ | 13 | 15 |

[a] Lateral flow assay (LFA); Chemiluminescence (CL); Molecularly imprinted polymer (MIP); Field-effect transistor (FET); Gold nanoparticles (AuNPs); Surface enhanced Raman scattering (SERS); Electrochemical paper-based analytical device (ePAD).

[b] Screen-printed electrode (SPE); Heat transfer method (HTM); Cyclic voltammetry (CV); Electrochemical impedance spectroscopy (EIS); Gold-thin film electrode (Au-TFE); Molecularly imprinted polymer with selectivity for the SARS-CoV-2 nucleocapsid protein (ncovNP-MIP); Differential pulse voltammetry (DPV); Antisense oligonucleotide (ASO); Surface plasmon resonance (SPR); Ribonuclease H (RNase H); Bioelectric recognition assay (BERA); Silver nanoparticles (AgNPs); Square wave voltammetry (SWV).

[c] Receptor binding domain (RBD).

#### Example 11: Use of NanoMIPs to detect SARS-CoV-2 in clinical samples

A prototype 3D-printed resin addition cell (Figure 11a and 11b) was developed for clinical analysis with disposable components. As in Example 10, the thermal measurement device was controlled using LabView software and a proportional-integral-derivative (PID) controller attached to a power resistor (22 $\Omega$) regulated the feedback on the signal. The PID parameters were optimized to reduce noise and were set at P = 1, $I$ = 14, and D = 0.

To validate the addition cell design, thermal detection experiments were performed using the SARS-CoV-2 spike protein in PBS. The addition cell sensor showed a good thermal response to the spike protein (Figure 11c) as the LoD value ($7.0 \pm 4.0$ fg mL$^{-1}$) was very similar to the results obtained when using the flow cell design ($9.9 \pm 2.5$ fg mL$^{-1}$ (Example 10)).

After initial validation, clinical measurements were performed using COVID-positive (positive after <20 PCR cycles) and negative patient samples (n = 7).

UTM and VPM (Viral preservation medium) were used as reference liquids for the negative and positive samples, respectively. During the measurements, 100 $\mu$L of the reference liquid (UTM/VPM) was pipetted into the reservoir and the $R_{th}$ signal was allowed to stabilize for 10 min. Following this, the reference liquid was pipetted out and 100 $\mu$L of the sample was added. Note that use of a pipette resulted in less disturbance to the system (e.g., flow, addition of air bubbles) compared to using a syringe pump. This is highly advantageous for clinical analysis since the sample volume was similar to that collected by a throat and nasal swab (100 $\mu$L), measurement time was reduced to ~15 min, and device operation was straightforward.

The thermal detection results (Figure 11d) show that specific binding to the nanoMIP cavities occurred during measurements of the positive samples, which led to a large mean $\Delta R_{th}$ value (0.27 °C W$^{-1}$). In contrast, only non-specific binding occurred to the nanoMIPs when measuring the negative samples, which resulted in a mean $\Delta R_{th}$ of 0.00 °C W$^{-1}$. The overall range of $\Delta R_{th}$ values was much larger for the positive samples (0.41 °C W$^{-1}$) compared to the negative samples (0.10 °C W$^{-1}$), which is due to variations in the viral loads of different COVID-positive patients. Importantly, there was no overlap in any positive and negative measurements as the largest $\Delta R_{th}$ for a negative sample was ~4 times lower than the smallest $\Delta R_{th}$ for a positive sample. This highlights that the nanoMIP sensor possesses excellent sensitivity and specificity for the detection of SARS-CoV-2 in clinical samples. Furthermore, the measurement time of the nanoMIP sensor (~15 mins) is comparable to commercial rapid antigen tests, which is crucially important for potential population screening applications.

#### Example 12: Analysis of binding between nanoMIPs and SARS-CoV-2 variants Alpha, Beta and Gamma.

NanoMIP synthesis

Peptide 1 was immobilised onto silanised glass beads prior to nanoMIP synthesis in line with the procedure in Example 1.

The peptide coated glass beads were used for the synthesis of imprinted nanoMIPs as follows: a monomer solution (see Example 1) was degassed under vacuum and sonicated for 5 min, and then purged with N$_2$ for 20 min before being added to 60 g of peptide 1-coated glass beads. Polymerization was initiated by adding an ammonium persulfate aqueous solution (800 $\mu$L, 60 mg/mL) and N,N,N',N'-tetramethylethylenediamine (24 $\mu$L), both from Sigma Aldrich. The headspace was flushed with N$_2$ and the bottle sealed with a screw cap. Polymerization was carried-out at room temperature for 1 h. Subsequently, the content of the polymerization vessel was poured into a solid-phase extraction (SPE) cartridge (60 mL) equipped with a frit (20 $\mu$m porosity). A total of 9 washes with 20 mL of distilled water at 20 °C were carried out to remove low affinity nanoMIPs, polymer and unreacted monomer. Afterwards, the SPE cartridge containing the solid-phase was placed in a water bath at 70 °C for 15 min. An aliquot of 20 mL of distilled water pre-warmed at 65 °C was poured into the SPE to collect the high-affinity nanoMIPs. This action was repeated 5 times, until about 100 mL of a solution of high-affinity nanoMIPs in water were collected. To ensure complete removal of any potential unreacted monomer from the bulk of the nanoparticles, the collected solution was concentrated down and dialysed with a SnakeSkin membrane (10 kDa molecular weight cut-off).

Imprinted nanoMIPs had an average diameter of 69.3nm, as calculated by NanoSight NS300 (Figure 12).

Sensor fabrication

Once produced the nanoMIPs imprinted against peptide 1 were integrated in a silver nanoparticle (AgNP) based localized surface plasmon resonance (LSPR) sensor.

In more detail, 50 μl of a nanoMIP stock solution (5 μg/ml in DI water) was dispensed on the Ag-LSPR chips. Afterwards, the Ag chips were placed inside a humidified chamber for 3h to ensure that the nanoMIPs were immobilized on the surface of the Ag nanoparticles. Since the nanoMIPs bear amine groups, electrostatic interactions between the negative LSPR Ag surface and the positively charged nanoMIPs (-Ag with NH3 +) resulted in binding of the nanoMIPs to the surface. After this the Ag substrates were thoroughly rinsed with DI water to remove any loosely-bound polymers from the electrode surface. The chips were then stored at 4°C.

Detection performance of nanoMIP - LSPR sensor.

The LSPR performance of the nanoMIP-LSPR sensor was then evaluated by detecting spike proteins of Alpha, Beta and Gamma Variants of the SARS-CoV-2 virus (all purchased from antibodies-online: Alpha, SARS-CoV-2 Spike protein lineage B.1.1.7, product number: ABIN6963738; Beta, SARS-CoV-2 Spike protein lineage B.1.351, product number: ABIN6963739; Gamma, SARS-CoV-2 Spike protein lineage P.1, product number: ABIN6964442) in PBS. For controls, the LSPR performance of the nanoMIP-LSPR sensor against spike proteins of human coronavirus strains HCoVOC43, HKU1 and HCoV-229E was tested as was the LSPR performance of a NIP-based LSPR sensor.

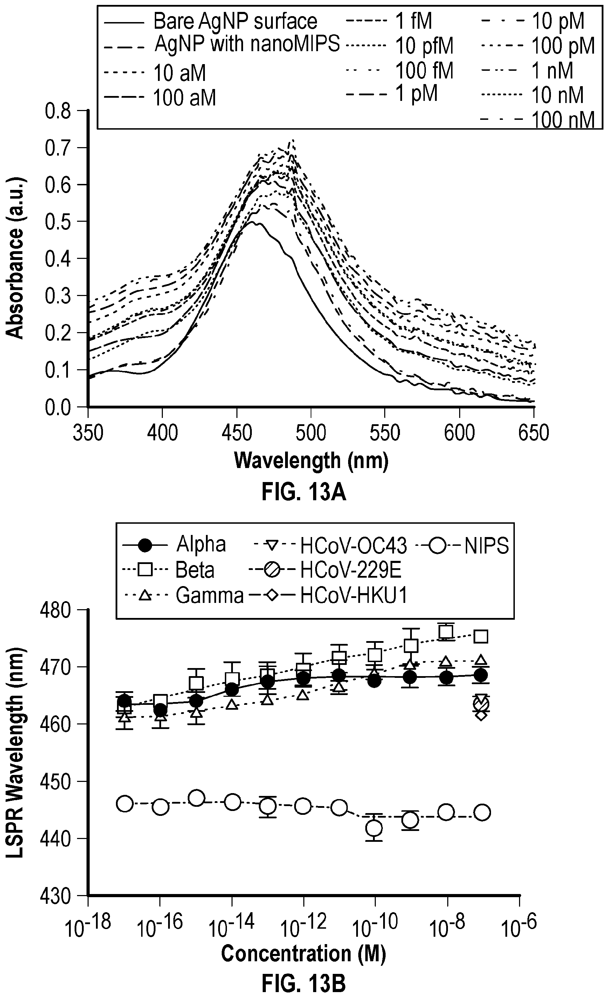

As shown in Figure 13, the sensor demonstrated successful detection of all SARS-CoV-2 variants. Figure 13a shows a typical LSPR sensor response upon binding of various concentrations of the alpha variant together with the corresponding absorbances. Figure 13b shows the change in LSPR wavelength of the Ag-MIPs complex upon varying the concentration of Alpha, Beta and Gamma from 10 aM to 100 nM.

The limit of detection (LOD) using wavelength data was found at 466.37 nm, 467.13 nm and 467.71 nm which corresponds to 9.71 fM, 7.32 fM and 8.81 pM respectively for Alpha, Beta and Gamma. The LOD was computed using empirical formula involving the use of limit of blank (LOB) and standard deviations of the measurements, where blank refers to the effect of PBS on the MIPs (without any proteins) - see below.

LOD calculation

To obtain the LOD of the LSPR sensor, the wavelength change was calculated using equation 1 ad 2 and then converted to the concentration of spike proteins of Alpha, Beta and Gamma variants.

$$\text{Limit of blank (LOB)} = \text{mean}_{blank} + 1.645 \, (SD_{blank}) \quad \text{(Eq. 1)}$$

$$\text{Limit of detection (LOD)} = \text{LOB} + 1.645 \, (SD_{lowest\ concentration}) \quad \text{(Eq. 2)}$$

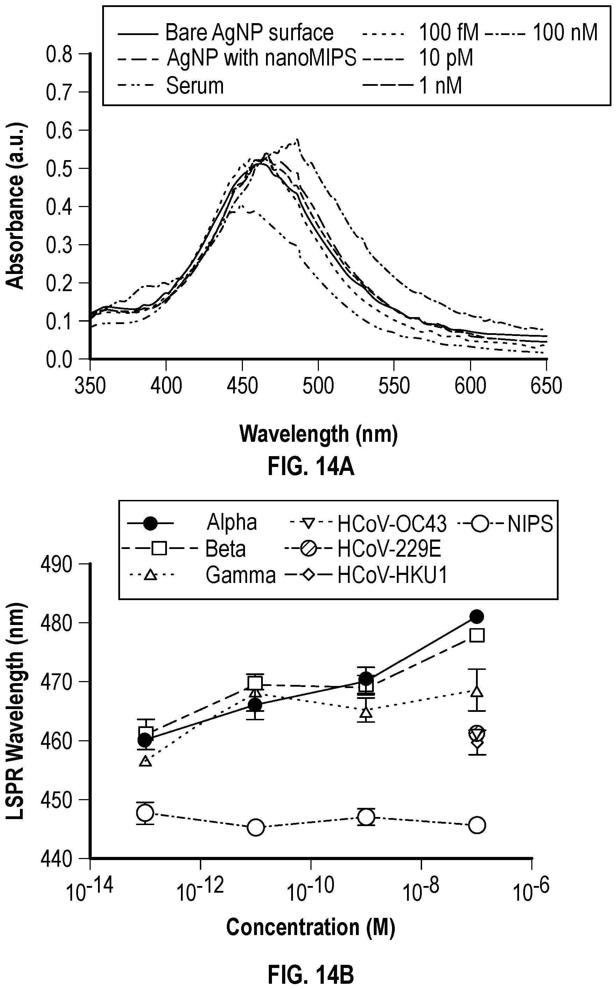

Following the successful detection of virus samples in PBS, similar experiments were conducted using the clinically relevant fluid human serum. In particular, serum was spiked with 100 fM, 10 pM, 1 nM and 100 nM concentrations of Alpha, Beta and Gamma spike protein and wavelength and absorbance shifts were measured. As for the above experiments using PBS, control experiments were also conducted using spike proteins of human coronavirus strains HCoVOC43, HKU1 and HCoV-229E and the LSPR performance of a NIP-based LSPR sensor was also tested. Figure 14a shows a typical LSPR sensor response to blood serum spiked with various concentrations of the Alpha spike protein. Figure 14b shows the change in LSPR wavelength of the Ag-MIPs complex upon varying the concentration of Alpha, Beta and Gamma spike proteins from 10 aM to 100 nM.

The LOD computed for Alpha and Beta using wavelength data was found to be at 457.54 nm and 460.49 nm. These LOD values correspond to concentrations of 14 fM for Alpha and 94 fM for Beta. The calculated LOD was 130 fM (at 457.37 nm) for wavelength changes observed for Gamma binding. The method for determining LOD values is shown above. Note that for LOB calculation, necessary for computing LOD, measurements in serum without protein were considered as blank samples in the present experiments.

For each of the above experiments in PBS and serum, all spike proteins, including those of the human coronavirus used as controls, were prepared or diluted with PBS/serum at different concentrations (10 aM to 100 nM). The Ag-MIP functionalised substrate was exposed to different concentrations of the respective protein. This was done by drop-casting 50 µl of the sample onto the Ag-MIPs. After exposing the surface of the sensor to a given concentration, a 20 min incubation period was included to allow the protein to interact with the MIPs. Thereafter, the surface of the sensor was washed with PBS (for both PBS and serum samples) and the LSPR signal was measured. To acquire the signal, a 30 s period was included during which the ocean view software (see below) acquired multiple spectrum and displayed an average of 10 spectrums with a box car width of 5. The LSPR spectra were later saved and wavelength/absorbance shifts were recorded, analyzed and plotted using Graphpad Prism software.

The LSPR signal was acquired using an in-house setup which consists of components purchased from Ocean Optics: spectrometer FLAME-T-XR1-ES, reflection probe QR400-7-SR-BX, UV-Vis patch connectors, DH-2000 Deuterium-Tungsten Halogen lamp (DH 2000-S-DUV-TTL), RTL-Tstage, and Ocean View software. Prior to the acquisition of the LSPR spectrum, dark and reference signals for background noise cancellation were measured using a glass slide as a reference. This glass slide was the same substrate on which Ag were deposited. This reference substrate was generated by complete removal of Ag nanoparticles from one of the substrates by sonicating the substrate in acetone for 1 hr and then using isopropanol wipes to clean the surfaces. All generated data were analysed and plotted using the in-built functionality of the GraphPad Prism 9 software.

### Example 13: Surface Plasmon Resonance (SPR) Analysis of NanoMIPs to SARS-CoV-2 spike proteins from two different SARS-CoV-2 variant strains.

A Biacore T200 instrument was employed in this study.

Surface activation and nanoMIP immobilisation.

NanoMIP-SPR sensors were prepared by covalently immobilising nanoMIPs (from Example 1) to CM5 chips from Cytiva in line with manufacturers guidelines.

In more detail, water was utilized to prime the instrument at a constant flow rate of 5µL/minute. Water was utilized as the running buffer throughout the chip preparation at a constant flow rate of 5µl/minute. Water was run until a stable baseline was achieved. A mixture of N-hydroxysuccinimide (NHS) and 1-ethyl-3-(3-dimethylaminopropyl)-carbodiimide (EDC) (2 mg EDC + 3 mg NHS in 400 uL of water) was injected (8 minutes at a flow rate of 5µL/minute) across the sensor surface to activate the surface (the mixture converts the carboxylic terminal groups on the chip surface into N-Hydroxy-succinimide esters). NanoMIPs (5nM) were then injected (7 minutes at a flow rate of 5µL/minute) into multiple flow cells on the chip. Finally, unreacted NHS esters deactivated by injecting ethanolamine in water.

Detection of interaction between nanoMIPs and SARS-CoV-2 spike proteins from two different SARS-CoV-2 variants

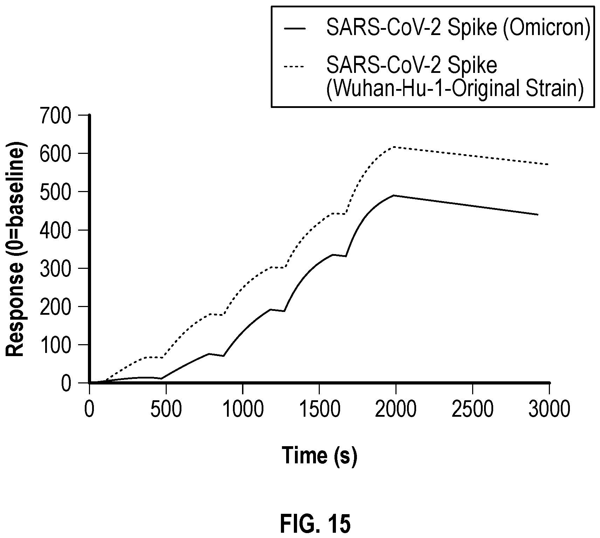

Once a stable baseline was obtained having switched running buffer from water to PBS, 5 dilutions (1.23, 3.7, 11.11, 33.33 and 100 nM in PBS) of recombinant SARS-CoV-2 S protein (recombinant full-length SARS-CoV-2 spike glycoprotein sourced from The Native Antigen Company, either designated as Wuhan Hu 1 original strain or Omicron strain) were injected (309 seconds at 5µl/minute) sequentially with minimal dissociation time only due to moving from one solution to the next. The results from the two spike protein variants were then superimposed such that the relative magnitude of binding response could be compared. As shown in Figure 15, the nanoMIPs continued to function despite the mutations in the spike protein in the Omicron strain, with only a small reduction in performance. Deriving a $K_D$ value was not deemed necessary.

### References

1. Wrapp et al., Science 367, 1260-1263 (2020)

2. van Grinsven, B.; Eersels, K.; Peeters, M.; Losada-Pérez, P.; Vandenryt, T.; Cleij, T. J.; Wagner, P. The Heat-Transfer Method: A Versatile Low-Cost, Label-Free, Fast, and User-Friendly Readout Platform for Biosensor Applications. ACS Appl. Mater. Interfaces 2014, 6, 13309-13318.

3. Geerets, B.; Peeters, M.; van Grinsven, B.; Bers, K.; de Ceuninck, W.; Wagner, P. Optimizing the Thermal Read-out Technique for MIP-Based Biomimetic Sensors: Towards Nanomolar Detection Limits. Sensors 2013, 13, 9148-9159.

4. Diagnostics Wantai SARS-CoV-2. WANTAI SARS-CoV-2 Ag Rapid Test (Colloidal Gold); 2020.

5. Grant, B. D.; Anderson, C. E.; Williford, J. R.; Alonzo, L. F.; Glukhova, V. A.; Boyle, D. S.; Weigl, B. H.; Nichols, K. P. SARS-CoV-2 Coronavirus Nucleocapsid Antigen Detecting Half-Strip Lateral Flow Assay toward the Development of Point of Care Tests Using Commercially Available Reagents. Anal. Chem. 2020, 92, 11305-11309.

6. Zhang, C.; Zhou, L.; Du, K.; Zhang, Y.; Wang, J.; Chen, L.; Lyu, Y.; Li, J.; Liu, H.; Huo, J.; Li, F.; Wang, J.; Sang, P.; Lin, S.; Xiao, Y.; Zhang, K.; He, K. Foundation and Clinical Evaluation of a New Method for Detecting SARS-CoV-2 Antigen by Fluorescent Microsphere Immunochromatography. Front. Cell. Infect. Microbiol. 2020, 10, 10.3389/fcimb.2020.553837.

7. Liu, D.; Ju, C.; Han, C.; Shi, R.; Chen, X.; Duan, D.; Yan, J.; Yan, X. Nanozyme Chemiluminescence Paper Test for Rapid and Sensitive Detection of SARS-CoV-2 Antigen. Biosens. Bioelectron. 2021, 173, 10.1016/j.bios.2020.112817.

8. Rahmati, Z.; Roushani, M.; Hosseini, H.; Choobin, H. Electrochemical Immunosensor with Cu2O Nanocube Coating for Detection of SARS-CoV-2 Spike Protein. Microchim. Acta 2021, 188, 10.1007/s00604-021-04762-04769.

9. Raziq, A.; Kidakova, A.; Boroznjak, R.; Reut, J.; Öpik, A.; Syritski, V. Development of a Portable MIP-Based Electrochemical Sensor for Detection of SARS-CoV-2 Antigen. Biosens. Bioelectron. 2021, 178, 10.1016/j.bios.2021.113029.

10. Seo, G.; Lee, G.; Kim, M. J.; Baek, S. H.; Choi, M.; Ku, K. B.; Lee, C. S.; Jun, S.; Park, D.; Kim, H. G.; Kim, S. J.; Lee, J. O.; Kim, B. T.; Park, E. C.; Kim, S. II. Rapid Detection of COVID-19 Causative Virus (SARS-CoV-2) in Human Nasopharyngeal Swab Specimens Using Field-Effect Transistor-Based Biosensor. ACS Nano 2020, 14, 5135- 5142.

11. Moitra, P.; Alafeef, M.; Alafeef, M.; Alafeef, M.; Dighe, K.; Frieman, M. B.; Pan, D.; Pan, D.; Pan, D. Selective Naked-Eye Detection of SARS-CoV-2 Mediated by N Gene 11 Targeted Antisense Oligonucleotide Capped Plasmonic Nanoparticles. ACS Nano 2020, 14, 7617-7627.

12. Mavrikou, S.; Moschopoulou, G.; Tsekouras, V.; Kintzios, S. Development of a Portable, Ultra-Rapid and Ultra-Sensitive Cell-Based Biosensor for the Direct Detection of the SARS-CoV-2 S1 Spike Protein Antigen. Sensors 2020, 20, 10.3390/s20113121.

13. Zhang, M.; Li, X.; Pan, J.; Zhang, Y.; Zhang, L.; Wang, C. Ultrasensitive Detection of SARS-CoV-2 Spike Protein in Untreated Saliva Using SERS-Based Biosensor. Biosens. Bioelectron. 2021, 190, 10.1016/j.bios.2021.113421.

14. Yakoh, A.; Pimpitak, U.; Rengpipat, S.; Hirankarn, N.; Chailapakul, O.; Chaiyo, S. PaperBased Electrochemical Biosensor for Diagnosing COVID-19: Detection of SARS-CoV-2 Antibodies and Antigen. Biosens. Bioelectron. 2021, 176, 10.1016/j.bios.2020.112912.

15. Fabiani, L.; Saroglia, M.; Galatà, G.; De Santis, R.; Fillo, S.; Luca, V.; Faggioni, G.; D'Amore, N.; Regalbuto, E.; Salvatori, P.; Terova, G.; Moscone, D.; Lista, F.; Arduini, F. Magnetic Beads Combined with Carbon Black-Based Screen-Printed Electrodes for COVID-19: A Reliable and Miniaturized Electrochemical Immunosensor for SARS-CoV2 Detection in Saliva. Biosens. Bioelectron. 2021, 171, 10.1016/j.bios.2020.112686.

## Sequence Summary

| SED ID NO: | Amino Acid Sequence |
|---|---|
| 1 | NSNNLDSKVGG |
| 2 | NYNYLYRLFRKS |
| 3 | YRLFRKSNLKPF |
| 4 | STEIYQAGSTPC |
| 5 | CNGVEGFNCYF |
| 6 | GSTPCNGVEGF |
| 7 | CYFPLQSYGFQP |
| 8 | GFQPTNGVGYQ |
| 9 | LQSYGFQPTNG |

## Claims

1. A molecularly imprinted polymer comprising at least one recognition site that is complementary to a template molecule consisting of an amino acid sequence corresponding to a subsequence of the receptor-binding domain of SARS-CoV-2 spike protein,

wherein the amino acid sequence is no more than 50 amino acids in length and comprises the sequence NSNNLDSKVGG, and

wherein the polymer comprises the monomers:

(i) N-Fluoresceinyl acrylamide,
(ii) Acrylamide,
(iii) Tert-Butyl acrylate (TBAc),
(iv) 3-O-Acryloyl-1,2:5,6-bis-O-isopropylidene-D-glucofuranose,
(v) 2,2,2-Trifluoroethyl methacrylate (CF3),
(vi) N-(3-Aminopropyl) methacrylamide hydrochloride (APMA),
(vii) Squareamide monomer 7 (SQ7) and
(viii) N,N'-Methylenebis(acrylamide) (BIS).

2. The molecularly imprinted polymer of claim 1, wherein the size of the polymer is less than 500nm.

3. The molecularly imprinted polymer of claim 1, wherein the size of the polymer is less than 250nm.

4. The molecularly imprinted polymer of claim 1, wherein the size of the polymer is less than 100nm.

5. A method of preparing a molecularly imprinted polymer comprising at least one recognition site which binds SARS-CoV-2 comprising the steps of:

(a) providing a carrier substance having a template molecule consisting of an amino acid sequence corresponding to a subsequence of the receptor binding domain of SARS-CoV-2 spike protein immobilised on it so as to be exposed at a surface;
(b) providing a polymerisable composition in contact with the surface;
(c) effecting controlled polymerisation of the polymerisable composition in contact with the surface to produce a molecularly imprinted polymer; and
(d) separating the molecularly imprinted polymer from the surface and the immobilised template molecule,

wherein the amino acid sequence is no more than 50 amino acids in length and comprises the sequence NSNNLDSKVGG, and

wherein the polymerisable composition comprises the monomers:

(i) N-Fluoresceinyl acrylamide,
(ii) Acrylamide,
(iii) Tert-Butyl acrylate (TBAc),
(iv) 3-O-Acryloyl-1,2:5,6-bis-O-isopropylidene-D-glucofuranose,
(v) 2,2,2-Trifluoroethyl methacrylate (CF3),
(vi) N-(3-Aminopropyl) methacrylamide hydrochloride (APMA),
(vii) Squareamide monomer 7 (SQ7) and
(viii) N,N'-Methylenebis(acrylamide) (BIS).

6. The method of claim 5, wherein the template molecule is modified at the N terminus to comprise an additional cysteine residue.

7. The method of claim 6, wherein the template molecule is modified so as to comprise a glycine residue between the cysteine residue and the last residue of the template.

8. A conjugate comprising:

(i) the molecularly imprinted polymer of any of claims 1-4 and
(ii) a fluorophore.

9. Use of (1) the molecularly imprinted polymer of any of claims 1-4, or (2) the conjugate of claim 8 in the detection of SARS-CoV-2.

**Patentansprüche**

1. Molekular geprägtes Polymer, umfassend mindestens eine Erkennungsstelle, die komplementär zu einem Template-Molekül ist, das aus einer Aminosäuresequenz besteht, die einer Teilsequenz der Rezeptor-bindenden Domäne des SARS-CoV-2-Spike-Proteins entspricht, wobei die Aminosäuresequenz nicht mehr als 50 Aminosäuren lang ist und die Sequenz NSNNLDSKVGG umfasst, und wobei das Polymer die folgenden Monomere umfasst:

   (i) N-Fluoresceinylacrylamid,
   (ii) Acrylamid,
   (iii) Tert-Butylacrylat (TBAc),
   (iv) 3-O-Acryloyl-1,2:5,6-bis-O-isopropyliden-D-glucofuranose,
   (v) 2,2,2-Trifluorethylmethacrylat (CF3),
   (vi) N-(3-Aminopropyl)methacrylamidhydrochlorid (APMA),
   (vii) Squareamidmonomer 7 (SQ7) und
   (viii) N,N'-Methylenbis(acrylamid) (BIS).

2. Molekular geprägtes Polymer nach Anspruch 1, wobei die Größe des Polymers kleiner als 500 nm ist.

3. Molekular geprägtes Polymer nach Anspruch 1, wobei die Größe des Polymers kleiner als 250 nm ist.

4. Molekular geprägtes Polymer nach Anspruch 1, wobei die Größe des Polymers kleiner als 100 nm ist.

5. Verfahren zum Herstellen eines molekular geprägten Polymers, das mindestens eine Erkennungsstelle umfasst, die SARS-CoV-2 bindet, umfassend die folgenden Schritte:

   (a) Bereitstellen einer Trägersubstanz, die ein Template-Molekül aufweist, das aus einer Aminosäuresequenz besteht, die einer Teilsequenz der Rezeptor-bindenden Domäne des SARS-CoV-2-Spike-Proteins entspricht, und das darauf immobilisiert ist, um an einer Oberfläche freigelegt zu werden;
   (b) Bereitstellen einer polymerisierbaren Zusammensetzung in Kontakt mit der Oberfläche;
   (c) Bewirken einer kontrollierten Polymerisation der polymerisierbaren Zusammensetzung in Kontakt mit der Oberfläche, um ein molekular geprägtes Polymer zu produzieren; und
   (d) Trennen des molekular geprägten Polymers von der Oberfläche und von dem immobilisierten Template-Molekül,
   wobei die Aminosäuresequenz nicht mehr als 50 Aminosäuren lang ist und die Sequenz NSNNLDSKVGG umfasst, und
   wobei die polymerisierbare Zusammensetzung die folgenden Monomere umfasst:

   (i) N-Fluoresceinylacrylamid,
   (ii) Acrylamid,
   (iii) Tert-Butylacrylat (TBAc),
   (iv) 3-O-Acryloyl-1,2:5,6-bis-O-isopropyliden-D-glucofuranose,
   (v) 2,2,2-Trifluorethylmethacrylat (CF3),
   (vi) N-(3-Aminopropyl)methacrylamidhydrochlorid (APMA),
   (vii) Squareamidmonomer 7 (SQ7) und
   (viii) N,N'-Methylenbis(acrylamid) (BIS).

6. Verfahren nach Anspruch 5, wobei das Template-Molekül an dem N-Terminus modifiziert ist, sodass es einen zusätzlichen Cysteinrest umfasst.

7. Verfahren nach Anspruch 6, wobei das Template-Molekül modifiziert ist, sodass es einen Glycinrest zwischen dem Cysteinrest und dem letzten Rest des Templates umfasst.

8. Konjugat, umfassend:

   (i) das molekular geprägte Polymer nach einem der Ansprüche 1-4 und
   (ii) einen Fluorophor.

9. Verwendung (1) des molekular geprägten Polymers nach einem der Ansprüche 1-4 oder (2) des Konjugats nach Anspruch 8 beim Nachweis von SARS-CoV-2.

**Revendications**

1. Polymère à empreinte moléculaire comprenant au moins un site de reconnaissance qui est complémentaire d'une molécule matrice constituée d'une séquence d'acides aminés correspondant à une sous-séquence du domaine de liaison au récepteur de la protéine de spicule du SARS-CoV-2,

ladite séquence d'acides aminés ne dépassant pas une longueur de 50 acides aminés et comprenant la séquence NSNNLDSKVGG, et
ledit polymère comprenant les monomères :

(i) N-fluoresceinyl acrylamide,
(ii) Acrylamide,
(iii) Acrylate de tert-butyle (TBAc),
(iv) 3-O-Acryloyl-1,2:5,6-bis-O-isopropylidène-D-glucofuranose,
(v) 2,2,2-Méthacrylate de trifluoroéthyle (CF3),
(vi) Chlorhydrate de N-(3-aminopropyl) méthacrylamide (APMA),
(vii) Monomère amide carré 7 (SQ7) et
(viii) N,N'-Méthylènebis(acrylamide) (BIS).

2. Polymère à empreinte moléculaire selon la revendication 1, la taille dudit polymère étant inférieure à 500 nm.

3. Polymère à empreinte moléculaire selon la revendication 1, la taille dudit polymère étant inférieure à 250 nm.

4. Polymère à empreinte moléculaire selon la revendication 1, la taille dudit polymère étant inférieure à 100 nm.

5. Procédé de préparation d'un polymère à empreinte moléculaire comprenant au moins un site de reconnaissance qui se lie au SARS-CoV-2 comprenant les étapes de :

(a) fourniture d'une substance porteuse comportant une molécule matrice constituée d'une séquence d'acides aminés correspondant à une sous-séquence du domaine de liaison au récepteur de la protéine de spicule du SARS-CoV-2 immobilisée sur celle-ci de manière à être exposée à une surface ;
(b) fourniture d'une composition polymérisable en contact avec la surface ;
(c) réalisation d'une polymérisation contrôlée de la composition polymérisable en contact avec la surface pour produire un polymère à empreinte moléculaire ; et
(d) séparation du polymère à empreinte moléculaire de la surface et de la molécule matrice immobilisée,
la séquence d'acides aminés ne dépassant pas une longueur de 50 acides aminés et comprenant la séquence NSNNLDSKVGG, et
la composition polymérisable comprenant les monomères :

(i) N-fluoresceinyl acrylamide,
(ii) Acrylamide,
(iii) Acrylate de tert-butyle (TBAc),
(iv) 3-O-Acryloyl-1,2:5,6-bis-O-isopropylidène-D-glucofuranose,
(v) 2,2,2-Méthacrylate de trifluoroéthyle (CF3),
(vi) Chlorhydrate de N-(3-aminopropyl) méthacrylamide (APMA),
(vii) Monomère amide carré 7 (SQ7) et
(viii) N,N'-Méthylènebis(acrylamide) (BIS).

6. Procédé selon la revendication 5, dans lequel la molécule matrice est modifiée à l'extrémité N pour comprendre un résidu cystéine supplémentaire.

7. Procédé selon la revendication 6, dans lequel la molécule matrice est modifiée de manière à comprendre un résidu glycine entre le résidu cystéine et le dernier résidu de la matrice.

8. Conjugué comprenant :

   (i) le polymère à empreinte moléculaire selon l'une quelconque des revendications 1 à 4 et
   (ii) un fluorophore.

9. Utilisation (1) du polymère à empreinte moléculaire selon l'une quelconque des revendications 1 à 4, ou (2) du conjugué selon la revendication 8 dans la détection du SARS-CoV-2.

FIG. 1A

FIG. 1B

FIG. 2

CPN510B+ MIP (COVID-19 P1-C6)                              ▽Detection Limit

| 290ng | 145ng | 73ng | 36ng | 18ng | 9ng | 5ng | 2ng | 1ng | 0.6ng | 0.3ng | 0.14ng |

CPN510B+ MIP (COVID-19 P3-C3)          ▽Detection Limit

| 290ng | 145ng | 73ng | 36ng | 18ng | 9ng | 5ng | 2ng | 1ng | 0.6ng | 0.3ng | 0.14ng |

CPN510B Only          ▽Detection Limit

| 290ng | 145ng | 73ng | 36ng | 18ng | 9ng | 5ng | 2ng | 1ng | 0.6ng | 0.3ng | 0.14ng |

FIG. 3

**CPN-MIP(COVID)**

**CPN-MIP(Troponin)**

Clear Spot with Spike Protein

Clear Spot with Virus

Clear Spot with Virus

No Spot without Virus / Spike Protein

**Nitrocellulose Membrane Layout**

| Peptide Positive Control | Virus Test |
|---|---|
| (A) | (B) |
| (C) | (D) |
| Virus Test | Culture Media Negative Control |

(E) CPN Reference Spot

20mm

**FIG. 4**

EP 4 392 177 B1

MIPs Vs Ab 1106

MIPs Vs Ab 1107

MIPs Vs Ab 5305

S1

OC43

229E

HKU1

**FIG. 5**

FIG. 6A

FIG. 6B

FIG. 7A

[RBD] (fg mL$^{-1}$)
Dynamic Range = 1 - 10,000 fg/mL
LoD = 2.55 ± 1.60 fg/mL

FIG. 7B

FIG. 8A

Dynamic Range = 1 - 10,000 fg/mL
LoD = 4.73 ± 0.92 fg/mL

FIG. 8B

FIG. 9A

FIG. 9B

FIG. 9C

FIG. 9D

FIG. 9E

FIG. 10A

FIG. 10B

FIG. 10C

**FIG. 10D**

**FIG. 10E**

FIG. 11A

FIG. 11B

FIG. 11C

FIG. 11D

FIG. 12

FIG. 13A

FIG. 13B

FIG. 14A

FIG. 14B

**FIG. 15**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021195626 A1

- US 10189934 B2

**Non-patent literature cited in the description**

- **CENNAMO et al.** *Proof of Concept for a Quick and Highly Sensitive On-Site Detection of SARS-CoV-2 by Plasmonic Optical Fibers and Molecularly Imprinted Polymers*
- **PARISI et al.** *Monoclonal-type" plastic antibodies for SARS-CoV-2 based on Molecularly Imprinted Polymers*
- **CUBUK et al.** *Computational analysis of functional monomers used in molecular imprinting for promising COVID-19 detection*
- **PILETSKY et al.** *Molecularly Imprinted Polymers for Cell Recognition*
- **MALIK et al.** *Molecular Imprinted Polymer for human viral pathogen detection*
- **WRAPP et al.** *Science*, 2020, vol. 367, 1260-1263
- **VAN GRINSVEN, B.** ; **EERSELS, K.** ; **PEETERS, M.** ; **LOSADA-PÉREZ, P.** ; **VANDENRYT, T.** ; **CLEIJ, T. J.** ; **WAGNER, P.** The Heat-Transfer Method: A Versatile Low-Cost, Label-Free, Fast, and User-Friendly Readout Platform for Biosensor Applications. *ACS Appl. Mater. Interfaces*, 2014, vol. 6, 13309-13318
- **GEERETS, B.** ; **PEETERS, M.** ; **VAN GRINSVEN, B.** ; **BERS, K.** ; **DE CEUNINCK, W.** ; **WAGNER, P.** Optimizing the Thermal Read-out Technique for MIP-Based Biomimetic Sensors: Towards Nanomolar Detection Limits. *Sensors*, 2013, vol. 13, 9148-9159
- *Diagnostics Wantai SARS-CoV-2. WANTAI SARS-CoV-2 Ag Rapid Test (Colloidal Gold)*, 2020
- **GRANT, B. D.** ; **ANDERSON, C. E.** ; **WILLIFORD, J. R.** ; **ALONZO, L. F.** ; **GLUKHOVA, V. A.** ; **BOYLE, D. S.** ; **WEIGL, B. H.** ; **NICHOLS, K. P.** SARS-CoV-2 Coronavirus Nucleocapsid Antigen Detecting Half-Strip Lateral Flow Assay toward the Development of Point of Care Tests Using Commercially Available Reagents. *Anal. Chem.*, 2020, vol. 92, 11305-11309

- **ZHANG, C.** ; **ZHOU, L.** ; **DU, K.** ; **ZHANG, Y.** ; **WANG, J.** ; **CHEN, L.** ; **LYU, Y.** ; **LI, J.** ; **LIU, H.** ; **HUO, J.** Foundation and Clinical Evaluation of a New Method for Detecting SARS-CoV-2 Antigen by Fluorescent Microsphere Immunochromatography. *Front. Cell. Infect. Microbiol.*, 2020, vol. 10
- **LIU, D.** ; **JU, C.** ; **HAN, C.** ; **SHI, R.** ; **CHEN, X.** ; **DUAN, D.** ; **YAN, J.** ; **YAN, X.** Nanozyme Chemiluminescence Paper Test for Rapid and Sensitive Detection of SARS-CoV-2 Antigen. *Biosens. Bioelectron.*, 2021, vol. 173
- **RAHMATI, Z.** ; **ROUSHANI, M.** ; **HOSSEINI, H.** ; **CHOOBIN, H.** Electrochemical Immunosensor with Cu2O Nanocube Coating for Detection of SARS-CoV-2 Spike Protein. *Microchim. Acta*, 2021, vol. 188
- **RAZIQ, A.** ; **KIDAKOVA, A.** ; **BOROZNJAK, R.** ; **REUT, J.** ; **ÖPIK, A.** ; **SYRITSKI, V.** Development of a Portable MIP-Based Electrochemical Sensor for Detection of SARS-CoV-2 Antigen. *Biosens. Bioelectron.*, 2021, vol. 178
- **SEO, G.** ; **LEE, G.** ; **KIM, M. J.** ; **BAEK, S. H.** ; **CHOI, M.** ; **KU, K. B.** ; **LEE, C. S.** ; **JUN, S.** ; **PARK, D.** ; **KIM, H. G.** Rapid Detection of COVID-19 Causative Virus (SARS-CoV-2) in Human Nasopharyngeal Swab Specimens Using Field-Effect Transistor-Based Biosensor. *ACS Nano*, 2020, vol. 14, 5135-5142
- **MOITRA, P.** ; **ALAFEEF, M.** ; **ALAFEEF, M.** ; **ALAFEEF, M.** ; **DIGHE, K.** ; **FRIEMAN, M. B.** ; **PAN, D.** ; **PAN, D.** ; **PAN, D.** Selective Naked-Eye Detection of SARS-CoV-2 Mediated by N Gene Targeted Antisense Oligonucleotide Capped Plasmonic Nanoparticles. *ACS Nano*, 2020, vol. 14, 7617-7627
- **MAVRIKOU, S.** ; **MOSCHOPOULOU, G.** ; **TSEKOURAS, V.** ; **KINTZIOS, S.** Development of a Portable, Ultra-Rapid and Ultra-Sensitive Cell-Based Biosensor for the Direct Detection of the SARS-CoV-2 S1 Spike Protein Antigen. *Sensors*, 2020, vol. 20

- **ZHANG, M.** ; **LI, X.** ; **PAN, J.** ; **ZHANG, Y.** ; **ZHANG, L.** ; **WANG, C.** Ultrasensitive Detection of SARS-CoV-2 Spike Protein in Untreated Saliva Using SERS-Based Biosensor. *Biosens. Bioelectron.*, 2021, vol. 190
- **YAKOH, A.** ; **PIMPITAK, U.** ; **RENGPIPAT, S.** ; **HIRANKARN, N.** ; **CHAILAPAKUL, O.** ; **CHAIYO, S.** PaperBased Electrochemical Biosensor for Diagnosing COVID-19: Detection of SARS-CoV-2 Antibodies and Antigen. *Biosens. Bioelectron.*, 2021, vol. 176
- **FABIANI, L.** ; **SAROGLIA, M.** ; **GALATÀ, G.** ; **DE SANTIS, R.** ; **FILLO, S.** ; **LUCA, V.** ; **FAGGIONI, G.** ; **D'AMORE, N.** ; **REGALBUTO, E.** ; **SALVATORI, P.** Magnetic Beads Combined with Carbon Black-Based Screen-Printed Electrodes for COVID-19: A Reliable and Miniaturized Electrochemical Immunosensor for SARS-CoV2 Detection in Saliva. *Biosens. Bioelectron.*, 2021, vol. 171